(19) Europäisches Patentamt
European Patent Office
Office européen des brevets

(11) EP 3 178 462 A1

(12) EUROPÄISCHE PATENTANMELDUNG

(43) Veröffentlichungstag:
14.06.2017 Patentblatt 2017/24

(51) Int Cl.:
A61K 6/02 (2006.01)      C04B 41/00 (2006.01)
C04B 41/45 (2006.01)      C04B 41/89 (2006.01)
C04B 41/52 (2006.01)      A61C 13/083 (2006.01)
B05C 1/00 (2006.01)       A61C 13/00 (2006.01)
A61K 6/00 (2006.01)       C04B 111/00 (2006.01)
C04B 111/82 (2006.01)

(21) Anmeldenummer: 16169366.8

(22) Anmeldetag: 12.05.2016

(84) Benannte Vertragsstaaten:
AL AT BE BG CH CY CZ DE DK EE ES FI FR GB
GR HR HU IE IS IT LI LT LU LV MC MK MT NL NO
PL PT RO RS SE SI SK SM TR
Benannte Erstreckungsstaaten:
BA ME
Benannte Validierungsstaaten:
MA MD

(30) Priorität: 07.12.2015 DE 102015121246

(71) Anmelder: WDT-Wolz-Dental-Technik GmbH
55566 Bad Sobernheim (DE)

(72) Erfinder: Wolz, Stefan
55566 Bad Sobernheim (DE)

(74) Vertreter: Götz, Georg Alois
Intellectual Property IP-GÖTZ
Patent- und Rechtsanwälte
Am Literaturhaus, Königstrasse 70
90402 Nürnberg (DE)

(54) **VERFAHREN ZUR HERSTELLUNG EINES POLYCHROM UND/ODER RÄUMLICH POLYCHROM ODER EINES MONOCHROM GEFÄRBTEN KERAMISCHEN KÖRPERS UND VORRICHTUNG HIERFÜR**

(57) Verfahren zur Herstellung eines polychrom und/oder räumlich polychrom oder eines monochrom gefärbten keramischen Körpers, insbesondere eines derart gefärbten Dentalkeramikrohlings, wobei zur Steuerung einer gezielten Verteilung von Farbpigmenten (101, 102) innerhalb einer porösen Keramik (100), in einem ersten Schritt, der ein Beladungsschritt (3c) ist, die Keramik (100) mit einer Farbpigmentlösung (104) beladen wird.

In einem zweiten Schritt, der ein Verteilungssteuerungsschritt (4d) ist, wird die Verteilung der Farbpigmente (101, 102) innerhalb der Keramik (100) durch Regelung eines oder mehrerer Umgebungsparameter (106) in einer Umgebung (108), insbesondere durch Regelung der Luftfeuchtigkeit und/oder des Drucks und/oder der Temperatur, gesteuert.

Fig. 12

EP 3 178 462 A1

**Beschreibung**

Die Erfindung betrifft ein Verfahren zur Herstellung eines polychrom und/oder räumlich polychrom oder eines monochrom gefärbten keramischen Körpers, wobei ein monochrom gefärbter Körper im Wesentlichen einfarbig ist, ein polychrom gefärbter Körper einen zweidimensionalen Farbverlauf aufweist und ein räumlich polychrom gefärbter beliebige Farbverläufe bzw. Farbverteilungen entlang beliebiger Raumrichtungen aufweist. Insbesondere betrifft die Erfindung ein Verfahren zur Herstellung eines derart gefärbten Dentalkeramikrohlings, der zur Bearbeitung mittels CAD/CAM geeignet ist.

In den letzten Jahren hat sich in der Dentaltechnik im Bereich der Vollkeramik die Verwendung von hydrostabilisiertem Zirkon durchgesetzt. Der ausschlaggebende Grund dafür ist die hohe Stabilität der Hochleistungskeramikgerüste. Die abschließende Farbgebung erfolgt bei vollkeramischem Zahnersatz durch das Aufbringen einer zusätzlichen Keramikschicht auf den bereits in Form gefrästen Zahnersatz, bspw. Kronen oder Brücken. Die zusätzliche Keramikschicht wird heute noch aufwendig und individuell für jeden einzelnen Zahnersatz von Hand aufgebracht. Eine maschinelle Herstellung ist nicht möglich. Jedoch wurde anhand von Studien nachgewiesen, dass die auf diese Weise hergestellte ästhetische Verblendkeramik, die exzellente Farbschichten und Formgebungsmöglichkeiten besitzt, eine fünfmal höhere Abplatzungsrate als die bewährte Verblendmetallkeramik (VMK) aufweist. Auf Grund häufiger Reklamationen und der hohen Herstellungskosten soll auf die ästhetische Verblendkeramik verzichtet werden. Ohne diese farbgebenden Möglichkeiten, die die Verblendkeramik besitzt, kann jedoch nur ein Zahnersatz hergestellt werden, der so gut wie keine Ästhetik aufweist.

Gemäß DE11 2009 001 253 WO 2008 098 157, WO 2013 055 432, WO 0046 168, WO 2004 110 959,DE 199 04 522 B4, DE 10 2008 026 980 A1, WO 00/46168 A1, WO 2011/156602 A2 oder DE 20 2011 109 956 U1, WO 11 15 66 02, EP 2013 06 31 20 wurden Metallionen- und/oder Metallkomplexlösungen und Gele zum Einfärben von poröser Dentalkeramik entwickelt, die einen einfarbigen Grundkörper eines Zahnes imitieren. Jedoch ist anzumerken, dass es keinen einfarbigen Zahn im Mund eines Patienten gibt. Dies wird auch in der DE 10 2008 026 980 A1 erklärt. An den Ecken eines Zahnes, wie zum Beispiel an einer Schneidekante, sammeln sich Farbpigmente, wodurch diese Ecken dunkler als der restliche Zahn wirken. Bei einem natürlichen Zahn wird der farbgebundene dunklere Dentinkern bis hin zu transparenten Zahnschneidekanten von einer immer dicker werdenden Schneidezahnmaße überzogen.

Durch das Eintauchen oder Besprühen des Zahnersatzes wird somit maximal eine einfache und einfarbige Färbung erreicht. Es wird zwar versucht mittels eines Pinsels oder Tropfenapplikationen Zahnfärbungsstrukturen oder ähnliche Einfärbungen auf dem Zahnersatz zu erzielen, jedoch lässt sich mit den bekannten Flüssigkeiten bzw. Lösungen oder Gelen kein naturidentisches Ergebnis erzielen, sodass sich zwischen natürlichen Zähnen und Zahnersatz merkliche farbliche Unterschiede ergeben. Des Weiteren ist aus der DE 199 04 522 B4 bekannt, dass unabhängig von der Einwirkungsdauer der Flüssigkeit bzw. der Lösung oder des Gels von 2 Minuten bis 20 Minuten immer der gleiche Farbton erreicht wird. Die Einwirkungstiefe ist außerdem entscheidend für die Farbentwicklung, vor allem beim Übergang in eine andere Farbe. Bei allen Lehren müssen spezifische langwirkende Trockenvorgänge vom Zahntechniker durchgeführt werden.

Ein weiterer Nachteil bei Flüssigkeiten bzw. Lösungen ergibt sich dadurch, dass deren Tropfen nicht lagebestimmt aufgetragen werden können und sich somit unkontrollierbar auf der durchgängig porösen Keramik verteilen. Es ist zum Beispiel nicht möglich nur die Kronenecken oder nur die Kronenspitzen einzufärben, da der farbgebende Tropfen genau an den Ecken oder Spitzen verläuft. Weiterhin wird mit den Auftragsinstrumenten, wie Malstiften und Pinseln Luft eingeschichtet bzw. kann Luft, die bereits innerhalb des porösen keramischen Zahnersatzes vorhanden ist beim Tauchen der porösen Keramik komplett eingegossen werden, so dass durch die eingeschlossene Luft es zu gar keiner Füllung der Porositäten und folglich auch zu keiner Einfärbung der Keramik kommen kann.

Ein Pinsel, der zum Auftragen der Farbe genutzt wird, hat zum Beispiel ein unkontrollierbares Depot an Einfärbungsflüssigkeit und/oder farbentfernende Lösungen und/oder farbentfernende Mittel und Gele. Es ist demnach ein Zufallsprinzip, wie viel Färbungs- und/oder farbentfernende Mittel der Pinsel aufgenommen hat. Ein Tropfen zuviel kann außerdem nicht vermieden noch korrigiert werden. Falls dies passiert, muss der komplette Zahnersatz neu angefertigt bzw. hergestellt werden. Alle Lehren beinhalten nur das Platzieren von farbgebenden Komponenten oder Entfernen der farbgebenden Komponenten.

Die Lösungen und Gele, die auf Wasser- und/oder Alkoholbasis aufgebaut sind, unterliegen einer schnellen Verdunstung. Die Verdunstung, die zum Beispiel durch falsche Lagerung entsteht, erhöht die Konzentration der Lösungen und Gele, so dass der Farbton ungewollt verändert wird. Des Weiteren können Lösungen beziehungsweise Flüssigkeiten oder Gele tropfen und auslaufen und somit Verschmutzungen, Verdunstungen und Verklebungen hinterlassen. Demnach ist das Tragen von Schutzkleidung, Schutzhandschuhen und Schutzbrille Vorschrift.

Damit eine Krone bzw. der Zahnersatz ästhetisch wirkt, sollten die im nachfolgenden aufgeführten Merkmale berücksichtigt und umgesetzt werden. Zum einen muss hierfür die Dentinschicht die Grundzahnfarbe des Patienten aufweisen. Die Dentinfarbe und die Schneideschichtstärke bilden die eigentliche Zahnfarbe des Patienten. Die dreidimensionale Farbkombination bildet der natürliche Zahn im Wachstum oder durch spätere Abnutzung. Zum anderen gibt es auch einen immer heller werdenden Farbverlauf vom Dentinkern bis hin zur Schneide. Der Zahnschmelz kann außerdem teilweise helle und/oder transparente Stellen aufweisen. Des Weiteren sind bei den älteren Patienten die dunklen dentinfarbenen Kronenränder gut sichtbar, so dass der neue Zahnersatz dementsprechend angepasst werden muss. Sollte der Zahnersatz nicht manuell individualisiert, sondern automatisiert hergestellt werden, müsste z.B. eine Vielzahl von ganz unterschiedlichen Keramikrohlingen hergestellt werden, um kostensenkende und ästhetische Ergebnisse zu erreichen.

Anders als bei der zuvor beschriebenen Herstellungsmethode wird nicht der herausgefräste Zahnersatz bzw. das poröse Zahnersatzgerüst, sondern bereits das Ausgangsmaterial, bspw. Keramikpulver oder -pasten zur Herstellung des Keramikrohlings gefärbt.

Diese Lösung schlagen die Lehren der EP 202 4300, WO 2014 062 375, WO 02 09 6 12, US 92 12 065 B2, DE 2020 090 187 24, EP 235 97 71 und EP 185 97 57 vor. Diese lehren das Einfärben des Ausgangsmaterials, insbesondere von Pulvern oder Pasten. Die Pulver oder Pasten werden schichtweise geschüttet bzw. aufgetragen, wobei jede Schicht eine konkrete Farbe aufweist. Somit sind 7-10 Schichten erforderlich, um eine zweidimensionale Farbgebung bzw. einen zweidimensionalen Farbverlauf zu erreichen. Die DE 2020 090 187 24, US 91 19 69 6, WO 02 09 6 12 lehren, dass diese Schichten gekrümmt werden, wodurch weitere Kosten für Spezialwerkzeuge, sowie für eine aufwendige Vielpulvereinbringung mit einer präzisen Dosiereinbringungsverteilung entstehen. Auch ist die Herstellung dieser Lehren auf Einzelkronen begrenzt. Des Weiteren zeigt die EP 18 59 75 7 die Schwierigkeiten auf, die an den Übergängen von Farbschicht zu Farbschicht entstehen. Sie lehrt, dass eine Schicht in den Übergangszonen mit Zwischenschichten auszulegen ist. Bei 7-10 Schichten multipliziert mit den 16 Grundzahnfarben ergeben sich hunderte von Pulver- und Pastenanmischungsmöglichkeiten, die jeweils mit hohen Kosten verbunden sind, Hunderte von Chargenprüfungs- und Kontrollkosten und enorme Lagerhaltungskosten. Auch werden die Rohlinge noch angesintert. Da die Sinteröfen aus Qualitätsgründen nur eine Zahnfarbe sintern können, kommt es zu weiteren Erhöhungen der Energiekosten. Ein Farbverlauf innerhalb einer Farbschicht von innen nach außen und somit ein naturidentischer, dreidimensionaler Farbverlauf lässt sich mit den beschriebenen Herstellungsverfahren nicht erzielen.

Es ist daher Aufgabe der Erfindung ein kostengünstiges, alternatives Herstellungsverfahren für einen monochrom, polychrom und/oder räumlich polychrom gefärbten keramischen Körper sowie eine Vorrichtung zu dessen Herstellung anzugeben.

Zur Lösung wird auf ein Verfahren gemäß Anspruch 1 verwiesen.

Das erfindungsgemäße Verfahren kennzeichnet sich dadurch, dass zur Steuerung einer gezielten Verteilung von Farbpigmenten in einem ersten Schritt, der ein Beladungsschritt ist, die poröse Keramik mit einer Farbpigmentlösung beladen wird. Eine Beladung ist im Allgemeinen als das Zuführen der Farbpigmente zu der porösen Keramik, vorzugsweise zu einer Oberfläche der porösen Keramik zu verstehen. Die Beladung kann insbesondere durch Auftragen von Farbpigmenten, vorzugsweise einer Farbpigmentlösung, mit einem Pinsel oder einem ähnlichen, geeigneten Applikationswerkzeug, aber auch mittels Einsprühen der porösen Keramik mit einer Farbpigmentlösung oder durch simples Eintauchen der porösen Keramik in eine Farbpigmentlösung erfolgen. Unter einer porösen Keramik, insbesondere in der Dentaltechnik, versteht man im Allgemeinen einen porösen, keramischen Körper der zum Beispiel durch Pressen aus Keramikpulver oder Schlickergießen aus keramischen Schlicker oder durch ähnliche, geeignete Verfahren geformt wurde. Um die Farbpigmente, z.B. metallische Ionen bzw. Metalloxide, oder auch andere organische oder anorganische Farbpigmente, gezielt innerhalb der porösen Keramik zu verteilen sind diese in einer flüssigen, insbesondere wässrigen Farbpigmentlösung enthalten. In einem zweiten Schritt, der ein Verteilungssteuerungsschritt ist, wird die Verteilung der Farbpigmente innerhalb der porösen Keramik gesteuert. D.h., die Bewegung der Farbpigmente wird derart gesteuert, dass diese an beliebige Positionen innerhalb der porösen Keramik transportiert werden. Hierzu werden ein oder mehrere Umgebungsparameter, insbesondere die Luftfeuchtigkeit und/oder der Druck und/oder die Temperatur in einer Umgebung, z.B. einem abgeschlossenen Gefäß, Schrank, Raum oder ähnlichem, in der sich die poröse Keramik befindet, geregelt. Unter einer Regelung wird nicht die ausschließlich quantitative Regelung sondern auch eine Regelung bezüglich festgelegter, örtlicher Bereiche innerhalb der Umgebung verstanden, d.h. die poröse Keramik kann bezüglich unterschiedlicher Oberflächen und/oder Oberflächenbereiche mit einem oder mehreren Umgebungsparametern beaufschlagt werden.

In einer vorteilhaften Verfahrensvariante wird die Verteilung der Farbpigmente innerhalb der porösen Keramik durch eine Konvektionsströmung bewirkt. Hierbei wird eine Strömungsrichtung und -geschwindigkeit, vorzugsweise der Farbpigmentlösung durch gezieltes Erzeugen von Umgebungsparametergradienten innerhalb der Umgebung gesteuert. Insbesondere werden Luftfeuchtigkeitsunterschiede und/oder Druckunterschiede und/oder Temperaturunterschiede bezüglich verschiedener Oberflächen und/oder bezüglich verschiedener Oberflächenbereiche der porösen Keramik eingestellt.

Gemäß einer vorteilhaften Ausführung des Verfahrens wird eine Bewegungsgeschwindigkeit der Farbpigmente und/oder die Strömungsgeschwindigkeit, insbesondere der Farbpigmentlösung, durch Erhöhen und/oder Verringern eines oder mehrerer der Umgebungsparametergradienten gesteuert. Beispielsweise kann ein erster Umgebungsdruck, der an einer ersten Oberfläche der porösen Keramik anliegt erhöht werden, und ein zweiter Umgebungsdruck, der an einer, der ersten Oberfläche gegenüberliegenden, zweiten Oberfläche der porösen Keramik anliegt verringert oder konstant gehalten werden, wodurch der Druckgradient bezüglich der beiden Oberflächen erhöht wird. Dies führt wiederum zu einer Änderung, insbesondere einer Erhöhung der Bewegungsgeschwindigkeit und/oder der Strömungsgeschwindigkeit.

Gemäß einer ebenso vorteilhaften Ausführung des Verfahrens wird eine Bewegungsrichtung der Farbpigmente und/oder die Strömungsrichtung, insbesondere der Farbpigmentlösung, durch Änderung der Richtung eines oder mehrerer der Umgebungsparametergradienten gesteuert. Beispielsweise kann der erste Umgebungsdruck an einer ersten Oberfläche der porösen Keramik angelegt werden und der zweite Umgebungsdruck an einer dritten Oberfläche der porösen Keramik, wodurch die Bewegungs- und/oder eine Strömungsrichtung hin zu einem Verlauf zwischen der ersten und der dritten Oberfläche der porösen Keramik geändert wird. Durch eine Umkehr des Umgebungsparametergradienten-Verlaufs (d.h. einer Änderung des Vorzeichens des Gradienten) kann die Bewegungsrichtung und/oder die Strömungsrichtung bspw. zwischen der ersten und der zweiten Oberfläche umgekehrt werden.

In einer optionalen Verfahrensvariante wird mindestens eine Oberfläche oder mindestens ein Teil einer Oberfläche der porösen Keramik während des Beladungsschritts und/oder während des Verteilungssteuerungsschritts gegenüber der Umgebung isoliert und/oder abgedichtet. Mindestens eine andere Oberfläche oder mindestens ein anderer Teil einer Oberfläche der porösen Keramik ist zur Beladung und/oder zur Steuerung frei zugänglich, d.h. steht mit der Umgebung in Verbindung. Auf diese Weise können die Umgebungsparameter bezüglich festgelegter Oberflächen und/oder Oberflächenteilbereiche örtlich geregelt werden. Die Isolierung und/oder Abdichtung kann während des Beladungsschritts zur örtlich gezielten Beladung der porösen Keramik mit den Farbpigmenten bzw. der Farbpigmentlösung und/oder während des Verteilungssteuerungsschritts zur gezielten Verteilung, insbesondere zur gezielten Steuerung der Bewegungsrichtung und/oder der Strömungsrichtung verwendet werden.

Vorteilhafterweise erfolgt die Beladung der porösen Keramik mit den Farbpigmenten, insbesondere mit der Farbpigmentlösung, über die mindestens eine, frei zugängliche Oberfläche.

Nach einer optionalen Verfahrensvariante wird die frei zugängliche Oberfläche der porösen Keramik vor der Beladung mit den Farbpigmenten und/oder mit der Farbpigmentlösung teilweise isoliert und/oder abgedichtet, sodass Teile dieser Oberfläche bzw. Oberflächenbereiche für die Beladung mit den Farbpigmenten bzw. der Farbpigmentlösung und/oder für die Beaufschlagung mit Umgebungsparametern unzugänglich sind. Vorteilhafterweise wird die frei zugängliche Oberfläche zur Beladung mit den Farbpigmenten und/oder mit der Farbpigmentlösung nach unten, in Richtung der Schwerkraft gerichtet.

Optional kann die mindestens eine, frei zugängliche Oberfläche der porösen Keramik, während des Verteilungssteuerungsschritts, mit der Umgebung in Verbindung stehen. Die frei zugängliche Oberfläche kann auf diese Weise mit einem oder mehreren der Umgebungsparameter beaufschlagt werden. Ebenfalls während des Verteilungssteuerungsschritts kann die mindestens eine isolierte und/oder abgedichtete Oberfläche gegen einen oder mehrere der Umgebungsparameter abgedichtet und/oder isoliert werden. Auf diese Weise können einzelne Bereiche der porösen Keramik gezielt mit Umgebungsparametern beaufschlagt werden, sodass Richtung und Ausprägungsgrad der Umgebungsparametergradienten gezielt eingestellt werden, um die räumliche Verteilung der Farbpigmente innerhalb der porösen Keramik zu steuern. Vorteilhafterweise erfolgt eine Abdichtung und/oder Isolierung mittels einer Form, eines Gehäuses oder ähnlichem und/oder einer Folie und/oder einer Beschichtung. Beispielsweise kann es sich bei der Form um eine Silikonform, bei der Folie um eine selbstklebende Folie und bei der Beschichtung um eine Silikon-, Latex- und/oder Wachsbeschichtung handeln.

Bei einer beispielhaften Verfahrensvariante wird die poröse Keramik zur Abdichtung und/oder Isolierung passgenau in eine Form, insbesondere eine teilgeöffnete Silikonform eingebracht, wobei der Druck innerhalb der Form geringer ist, als bspw. der Umgebungsdruck. In einem Ausführungsbeispiel des Verfahrens ist mindestens eine erste Oberfläche und/oder ein erster Oberflächenteilbereich der porösen Keramik innerhalb der Form angeordnet, wobei diese erste Oberfläche und/oder dieser erste Oberflächenteilbereich zumindest gegenüber einem Umgebungsparameter, bspw. gegenüber der Luftfeuchtigkeit oder dem Druck die bzw. der in der Umgebung vorliegt, isoliert und/oder abgedichtet ist. Mindestens eine zweite Oberfläche und/oder ein zweiter Oberflächenteilbereich ist außerhalb der Form angeordnet, wobei diese zweite Oberfläche und/oder dieser zweite Oberflächenteilbereich bezüglich der Umgebungsparameter frei zugänglich ist. Mindestens eine dritte Oberfläche und/oder ein dritter Oberflächenteilbereich ist innerhalb der Form angeordnet und liegt an einer Innenwandung der Form an, sodass innerhalb der Form bspw. ein Unter- oder Überdruck gegenüber dem Umgebungsdruck einstellbar ist. Beispielsweise kann ein Druckunterschied zwischen der innenliegenden, ersten Oberfläche und der außenliegenden, zweiten Oberfläche der porösen Keramik eingestellt werden, indem der Druck innerhalb der Form geringer ist als der Umgebungsdruck. Zur Umkehr der Richtung des Druckgradienten kann der Druck innerhalb der Form erhöht oder alternativ der Umgebungsdruck erniedrigt werden, bis der Druck innerhalb der Form höher ist als der an der zweiten, frei zugänglichen Oberfläche anliegende Umgebungsdruck. Alternativ kann die poröse Keramik innerhalb der Form gewendet werden, sodass die erste Oberfläche frei zugänglich außerhalb der Form angeordnet ist und die zweite Oberfläche isoliert und/oder abgedichtet innerhalb der Form angeordnet ist. Optional kann die poröse Keramik mit zusätzlichen Umgebungsparametergradienten, wie beispielsweise Luftfeuchtigkeitsunterschieden beaufschlagt werden, wobei die Luftfeuchtigkeit vorzugsweise an der zweiten, frei zugänglichen Fläche geregelt wird. Durch die Verwendung von selbstklebender Folie auf der zweiten, frei zugänglichen Oberfläche kann ein Umgebungsparameter, wie die Luftfeuchtigkeit bezüglich einzelner, zweiter Oberflächenteilbereiche geregelt werden.

Vorteilhafterweise enthält das Lösungsmittel der Farbpigmentlösung Wasser und Zirkon-Nitrat. Über die Menge des zugegebenen Zirkon-Nitrats bzw. den Anteil an Zirkon-Nitrat in der Farbpigmentlösung lässt sich die Intensität und/oder die Sättigung der einzelnen Farben bzw. der Farbausprägungsgrad, der im gefärbten keramischen Körper erhalten werden soll steuern.

Optional kann die Beladung der porösen Keramik mit den Farbpigmenten bzw. mit der Farbpigmentlösung mittels eines Beladungskörpers erfolgen. Insbesondere umfasst der Beladungskörper ein poröses und/oder schwammartiges Material, das die Aufnahme der Farbpigmente bzw. der Farbpigmentlösung mit den darin enthaltenen Farbpigmenten begünstigt. Der Beladungskörper ist hierbei mit einem Lösungsmittel und darin enthaltenen Farbpigmenten versetzt, insbesondere gesättigt. Vorzugsweise wird die poröse Keramik zur Beladung mit den Farbpigmenten, bzw. mit der Farbpigmentlösung während des Beladungsschritts mit einer frei zugänglichen Oberfläche auf den Beladungskörper aufgelegt. Die optionale Beladungsmethode bietet den Vorteil, dass die gesamte, frei zugängliche Oberfläche der porösen Keramik mit dem Beladungskörper in berührender Verbindung steht, wodurch diese gleichmäßig mit Farbpigmenten beaufschlagt wird. Die Anzahl der Farbpigmente und/oder die Farbpigmentkonzentration pro Fläche, die der porösen Keramik während des Beladungsschritts zugeführt werden, lässt sich auf diese Weise über die gesamte, frei zugängliche Fläche konstant halten.

Der Beladungskörper kann beispielsweise eine oder mehrere Schichten umfassen wobei die eine oder die mehreren Schichten gleiche Farbpigmente zur Herstellung einer monochromen Keramik enthalten. Eine monochrome Keramik bzw. ein monochrom gefärbter, keramischer Körper kann hergestellt werden, indem bspw. alle Schichten des Beladungskörpers mit gleichen Farbpigmenten, d.h. mit der gleichen Farbe versetzt und/oder gesättigt werden. Um eine polychrome Keramik bzw. einen polychrom gefärbten, keramischen Körper herzustellen, können verschiedene Schichten mit verschiedenen Farbpigmenten, d.h. mit unterschiedlichen Farben versetzt und/oder gesättigt werden. Die mehreren Schichten des Beladungskörpers können horizontal nebeneinander und/oder vertikal untereinander bzw. übereinander angeordnet sein. Mittels vertikal angeordneter Schichten, wird bereits bei der Beladung ein vertikaler, polychromer Farbverlauf innerhalb des keramischen Körpers begünstigt oder erzeugt. Die zu beladende, poröse Keramik steht lediglich mit der obersten Schicht des Beladungskörpers in Verbindung bzw. liegt auf der obersten Schicht des Beladungskörpers auf. Die Farbpigmente, die in den übrigen Schichten enthalten sind, durchlaufen die jeweils oberhalb angeordneten Schichten, bevor sie in die poröse Keramik eindringen. Auf diese Weise lässt sich eine zeitlich versetzte Beladung mit verschiedenen Farbpigmenten, die in einem weichen Farbverlauf resultiert, erzielen. Eine horizontale Anordnung der Schichten, kann bereits während der Beladung der porösen Keramik einen horizontalen, polychromen Farbverlauf innerhalb des keramischen Körpers begünstigen oder erzeugen. Die zu beladende, poröse Keramik steht hierbei mit allen horizontal nebeneinander angeordneten Schichten in Verbindung bzw. liegt auf diesen auf, sodass die Farbpigmente der einzelnen Schichten gleichzeitig, jedoch an verschiedenen Oberflächenteilbereichen der frei zugänglichen Oberfläche in die poröse Keramik eindringen. Eine Kombination von vertikal über- bzw. untereinander und horizontal nebeneinander angeordneten Schichten begünstigt oder erzeugt bereits bei der Beladung einen dreidimensionalen bzw. räumlichen, polychromen Farbverlauf innerhalb des keramischen Körpers. Je nach gewünschtem Endergebnis kann die Kombination von Schichten horizontal nebeneinander und vertikal über- bzw. untereinander beliebig erfolgen.

Natürlich können unterschiedliche Farbpigmente zur Herstellung eines polychrom oder räumlich polychrom gefärbten keramischen Körpers auch nacheinander, in mehreren Beladungsschritten, mit jeweils einer einzigen Schicht des Beladungskörpers der porösen Keramik zugeführt werden.

Nach einer optionalen Verfahrensvariante umfasst der Beladungskörper einen Filter. Der Filter bildet vorzugsweise die oberste Schicht des Beladungskörpers und kann mit dem Lösungsmittel, ohne Farbpigmente versetzt bzw. gesättigt sein. Durch die Verwendung des Filters der insbesondere ein gleiches oder ähnliches Material wie die übrigen Schichten des Beladungskörpers umfasst, kann eine gleichmäßigere Verteilung der Farbpigmente während des Beladungsschritts und somit eine einfachere Steuerung des Farbverlauf erzielt werden. Insbesondere enthält der Filter ein mit Zirkon-Nitrat versetztes Lösungsmittel.

Vorteilhafterweise erfolgt eine Trocknung der porösen Keramik, insbesondere eine vollständige Trocknung bereits während des Verteilungssteuerungsschritts. Beispielsweise können die Umgebungsparameter, insbesondere die Luftfeuchtigkeit und/oder der Druck und/oder die Temperatur zur Steuerung des Trocknungsvorgangs geregelt werden, wodurch einerseits die Trocknungszeit, andererseits der örtliche Trocknungsverlauf gezielt variiert werden kann. So kann eine Trocknung insbesondere von einer, mehreren oder sämtlichen frei zugänglichen Oberflächen der porösen Keramik bzw. des keramischen Körpers aus erfolgen. Insbesondere lassen sich die Farbpigmente durch Trocknung, d.h. Verdunstung des Lösungsmittels ortsfest innerhalb der porösen Keramik an der gewünschten Position fixieren.

Alternativ oder optional kann die poröse Keramik bzw. der keramische Rohling in einem zusätzlichen Trocknungsschritt wärmebehandelt werden, der sich an den Verteilungssteuerungsschritt bzw. an die Verteilung der Farbpigmente innerhalb der porösen Keramik anschließt. Die poröse Keramik oder der Keramikrohling wird hierbei zur Ausbildung einer Oxidphase, insbesondere einer Nitratoxidphase einer Temperatur in einem Bereich zwischen 80°C und 1200°C, vorzugsweise zwischen 80°C und 800°C ausgesetzt. Mit Sauerstoff bilden in Lösung befindliche Kationen bspw. von Salzen, insbesondere Metallsalzen, die als Farbpigmente dem Lösungsmittel beigesetzt sind, Oxide bzw. eine Oxidphase. Insbesondere wird der poröse, keramische Körper einer Wärmebehandlung ausgesetzt um einerseits eine Fixierung, und andererseits eine Entwicklung der Farbpigmente zu erreichen. So bildet ein gelöstes metallisches Kation mit Sauerstoff ein Metalloxid, ein sogenanntes Metalloxid-Pigment, welches die Färbung der Keramik bedingt. Durch die Zugabe von Zirkon-Nitrat zu dem Lösungsmittel bzw. der Farbpigmentlösung sowie einen Trocknungsschritt mit Wärmebehandlung lassen sich die Farbpigmente zuverlässig an der gewünschten Position fixieren. Aufgrund der Wärmebehandlung bildet das Zirkon-Nitrat eine feste Struktur innerhalb der Poren der porösen Keramik, welche die Farbpigmente einschließt bzw. fixiert. Die Wärmebehandlung ermöglicht insbesondere ein Fräsen mit wasserkühlenden CAD/CAM-Maschinen, ohne dass eine Delokalisierung bzw. eine Verschiebung der Pigmente innerhalb der porösen Keramik zu befürchten ist.

Während und/oder nach der Verteilung der Farbpigmente innerhalb der porösen Keramik kann gemäß einer optionalen Verfahrensvariante ein WAK-Ausgleich durchgeführt werden, wobei die poröse Keramik zumindest teilweise mit einem Ausgleichsstoff beladen wird. Zur Fertigstellung eines Zahnersatzes wird der keramische Rohling üblicherweise einem Endsintervorgang, d.h. einer Hochtemperaturbehandlung ausgesetzt, wodurch eine Werkstoffverdichtung erzielt wird und die Porenräume aufgefüllt werden. Aufgrund unterschiedlicher Wärmeausdehnungskoeffizienten (WAK) der verschiedenen Materialien, bspw. des Keramikrohlings sowie der infiltrierten Farbpigmente können Spannungen durch voneinander abweichende, wärmebedingte Volumenausdehnungen auftreten. Derlei Spannungen führen häufig zu einer Rissbildung, wodurch der keramische Körper als Zahnersatz unbrauchbar wird. Durch Zugabe eines Ausgleichsstoffs werden die unterschiedlichen WAK-Werte angeglichen und Spannungen sowie Rissbildungen vermieden.

Gemäß einem beispielhaften, erfindungsgemäßen Verfahrensablauf wird in einem ersten Schritt ein im Wesentlichen flacher und/oder plattenförmiger, poröser Keramikrohling, insbesondere zur Verwendung im Dentalbereich geeignet, bereitgestellt. Plattenförmige Dental-Keramikrohlinge sind im Handel erhältlich und eignen sich zur Verarbeitung mit einer herkömmlichen CAD/CAM-Keramikfräsmaschine und zur anschließenden Endsinterung zur Herstellung von Zahnersatz. In einem zweiten Verfahrensschritt werden eine oder mehrere Oberflächen des Keramikrohlings mit einer Isolierung und/oder Abdichtung versehen, wobei der Keramikrohling in einer wasser- und luftundurchlässigen, d.h. dichtenden Form, insbesondere einer Silikonform platziert wird. Hierbei wird mindestens eine Oberfläche oder ein Oberflächenteilbereich des Keramikrohlings nicht von der Form isoliert und/oder abgedichtet, sodass diese Oberfläche zur Beaufschlagung mit Umgebungsparametern bzw. zur Beladung frei zugänglich ist. In einem dritten Schritt, einem Beladungsschritt, wird die frei zugängliche Oberfläche bzw. der frei zugängliche Oberflächenbereich des Keramikrohlings mit Farbpigmenten beladen, wobei die Farbpigmente, bspw. metallische Salze, in einer flüssigen, insbesondere wässrigen Lösung enthalten sind bzw. in Lösung vorliegen. Der Keramikrohling wird in einem vierten Schritt innerhalb einer Umgebung, deren Umgebungsparameter, insbesondere die Luftfeuchtigkeit und/oder der Druck und/oder die Temperatur regelbar sind, platziert. Hierbei kann es sich beispielsweise um einen Klimaschrank oder Trockenschrank, aber auch einen begehbaren Raum, dessen Umgebungsparameter regelbar sind, handeln. Der Keramikrohling kann hierbei in der Form belassen werden, sodass lediglich die frei zugängliche Oberfläche mit der Umgebung in Verbindung steht. In einem fünften Schritt, einem Verteilungssteuerungsschritt, wird die Verteilung der Farbpigmente, die mittels der Beladung mit der Farbpigmentlösung in den Keramikrohling eingebracht wurden, innerhalb des Keramikrohlings gesteuert. Hierzu wird mindestens ein Umgebungsparameter, insbesondere die Luftfeuchtigkeit und/oder der Druck und/oder die Temperatur zur Erzeugung eines Umgebungsparametergradienten zwischen der einen oder den mehreren frei zugänglichen Oberflächen und der einen oder den mehreren isolierten und/oder abgedichteten Oberflächen des Keramikrohlings geregelt.

In einem optionalen, zusätzlichen Verfahrensschritt kann die poröse Keramik oder der Keramikrohling nach der Verteilung und gegebenenfalls Fixierung der Farbpigmente mittels einer CAD/CAM-Fräsmaschine in die gewünschte Form, insbesondere in die Form eines Zahnersatzteils gefräst werden. In einem weiteren, optional zusätzlichen Verfahrensschritt kann die poröse Keramik oder der Keramikrohling nach der Verteilung und gegebenenfalls Fixierung der Farbpigmente, sowie vorzugsweise nach einer formgebenden Bearbeitung wie beispielsweise Fräsen, gesintert bzw. endgesintert werden. Insbesondere kann der Keramikrohling vor der Verteilung der Farbpigmente bei niedrigerer Temperatur angesintert und nach der Verteilung der Farbpigmente bei höherer Temperatur endgesintert werden.

Die Erfindungsaufgabe wird ebenfalls gelöst durch einen Keramikrohling, der zur Herstellung von Zahnersatz mittels einer CAD/CAM-Fräsmaschine geeignet ist und insbesondere nach einem erfindungsgemäßen Verfahren hergestellt wurde. Der Keramikrohling weist eine Farbpigment-Verteilung auf, die mittels Umgebungsparametergradienten steuerbar ist, wobei der Keramikrohling eine gleichmäßige, monochrome Färbung und/oder einen polychromen Farbverlauf und/oder einen räumlich, polychromen Farbverlauf aufweist.

Eine erfindungsgemäße Vorrichtung zur Beladung einer porösen Keramik, insbesondere eines Dental-Keramikrohlings mit einer wässrigen Farbpigmentlösung kennzeichnet sich dadurch, dass die Beladungsvorrichtung ein poröses und/oder schwammartiges Material umfasst. Das poröse und/oder schwammartige Material ist mit der Farbpigmentlösung versetzbar, vorzugsweise sättigbar, sodass diese an die beispielsweise aufliegende, poröse Keramik abgebbar ist bzw. die poröse Keramik mit dieser beladbar ist. Gemäß einer vorteilhaften Ausführung umfasst die Beladungsvorrichtung mindestens zwei Schichten, wobei mindestens eine Schicht als Filter ausgebildet, d.h. nicht mit Farbpigmenten versetzt ist und/oder mindestens zwei Schichten mit jeweils verschiedenen Farbpigmenten versetzt sind.

Eine erfindungsgemäße Vorrichtung zur Steuerung einer gezielten Verteilung von Farbpigmenten innerhalb einer porösen Keramik, insbesondere eines Dental-Keramikrohlings ist zur Isolierung und/oder Abdichtung von mindestens einer Oberfläche und/oder mindestens eines Teils einer Oberfläche der porösen Keramik geeignet, wobei mindestens eine andere Oberfläche und/oder mindestens ein anderer Teil einer Oberfläche zur Beaufschlagung mit regelbaren Umgebungsparametern frei zugänglich ist. Beispielsweise handelt es sich bei der Vorrichtung um eine geöffnete Form, insbesondere eine Silikonform, die zur passgenauen Aufnahme der porösen Keramik ausgebildet ist, sodass die poröse Keramik formschlüssig von der Silikonform aufgenommen wird, wobei eine Oberfläche der porösen Keramik frei zugänglich ist.

Den nachfolgenden Abschnitten sind weitere beispielhafte Merkmale, Merkmalskombinationen und Ausgestaltungen im Rahmen der Erfindung zu entnehmen.

In der Regel lassen sich die entsprechenden Lösungen in einfacher Weise dadurch herstellen, dass ein entsprechendes z.B. Metallsalz in den entsprechenden Lösungsmitteln, vorzugsweise Wasser aufgelöst wird.

Vorzugsweise wird bei der Erfindung dabei von den entsprechenden Salzen wie Chloraten, Sulfaten, Carbonaten oder insbesondere Nitraten des z.B. jeweiligen Metalls ausgegangen. Die Seltenen Erdenelemente umfassen, bekanntlich auch insbesondere die Gruppen der Lanthanoide. Bei den Nebengruppenelementen sind insbesondere die Übergangsmetalle hervorzuheben, sowie 1-8 Nebengruppe, I-VIII Hauptgruppe. Nach neueren Nomenklatur des Periodensystems der Elemente.

Der verwendbare Ausdruck "Lösung" ist dem Fachmann ohne weiteres bekannt und sollte hier möglichst umfassend verstanden werden. Selbstverständlich werden die Metallionen bzw. Metallkomplexe erfindungsgemäß in einer Form bereitgestellt, in der sie möglichst leicht in das poröse Keramikmaterial eindringen können. Deshalb wird es sich hier in der Regel eine (flüssige) Lösung bzw. um ein homogenes Gemisch eines entsprechenden Feststoffen in der Lösung um die Porositäten des Keramikkörpers zu beladen. Nach Trocknung der Nitrate in den Porositäten entwickelt sich ein Kristall, das durch eine gesonderte Wärmebehandlung auch eine Oxidationsstufe durchlaufen kann.

Bei dem erfindungsgemäßen Verfahren, die besonders bevorzugt einsetzbar sind, Suspension oder insbesondere Lösungen, die Metallionen der Metallkomplexe mit mindestens einem Element der aufgeführten Elemente:

1. $Fe(No_3)_3 \cdot 9\ H_2O$

2. $Cr((No_3)_3 \cdot 9\ H_2O$

3. $Er(No_3)_3 \cdot 5\ H_2O$

4. $Ce(No_3)_3 \cdot 6\ H_2O$

5. $Al\ (No_3)_3 \cdot 9\ H_2O$

6. $Ni(No_3)_2 \cdot 6\ H_2O$

7. $Mn(No_3) \cdot 4\ H_2O$

8. $Pr(No_3)_3 \cdot 6\ H_2O$

9. $Y(No_3)_3 \cdot 6\ H_2O$

10. $Co(No_3)_2 \cdot 6\ H_2O$

11. $ZrO(No_3)_2 \cdot x\ H_2O$

12. $Sm(No_3)_3 \cdot 6\ H_2O$

13. $Nd(No_3)_3 \cdot 6\ H_2O$

14. $Eu(No_3)_3 \cdot 5\ H_2O$

15. $Dy(No_3)_3 \cdot x\ H_2O$

16. $Yb(No_3)_3 \cdot 5\ H_2O$

17. $Ti(No_3)_4 \cdot 4\ H_2O$

18. $Bi(No_3)_3 \cdot 5\ H_2O$

19. Au Ci

20. $Sr(No_3)_2$

21. $Mg(No_3)_2 \cdot 6\ H_2O$

22. $La(No_3)_3 \cdot 6\ H_2O$

23. $Ag\ NO_3 \cdot$

24. $In(No_3)_3 \cdot X\ H20$

25. $Cd(No_3)_2 \cdot 4\ H_2O$

26. $V(No_3)$

27. $Zn(No_3)_2 \cdot 6\ H_2O$

28. $Dy(No_3)_3 \cdot xH_2O$

29. $Tb(No_3)_3 \cdot 5\ H20$

30. $Ca(No_3)_2 \cdot xH_2O$

31. $C_4H_4NNbo_9 \cdot x\ H_2O$

32. $Pb(No_3)_2$

33. $Nb(No_3)_3 \cdot 5H_2O$

34. $Hf(No_3)_4$

35. $Zr\ (So_4)_2 \cdot H_2O$

enthalten.

Bei dem vorgeschlagenen Verfahren zum Färben von gebinderter und/oder entbinderter und/oder angesinterter und/oder durchgängig poröser Keramik, insbesondere von porösen Körpern, die insbesondere in der Dentaltechnik Anwendung finden, erfolgt die farbgebende Verteilung in Keramikkörper und/oder auch in verkaufsfertigen Keramikrohlingen in einem dichten z.B. Silikongehäuse, um auch die Bewegungsrichtung der Farbpigmente gesteuert vorzugeben. In dem z.B. Silikongehäuse befindet sich auch ein Raum mit und/oder ohne sphärischen Druck mit und/oder Beladungskörperstoffe zur luftfreien kontinuierlichen Befüllung der Porositäten. Unter einem Abdichtung- und/oder Isolierungsmittel einer Form versteht man alle Materialien die Flächen eines porösen Keramikrohlings dicht oder luftdurchlässig und/oder umgeben können. Vorzugsweise wird eine Fläche des porösen Keramikrohlings auf einen kapillardruckhaltenden Beladungskörperstoffe Lösungsmittelvorrichtung aufgesetzt, in der oder auf der Farbpigmentlösungen gespeichert sind. Vorteilhaft ist die Auflage zum luftfreien Transport aus Beladungskörperstoffen herzustellen, wie Mikrofaser, Schwämme, Zellstoff usw. Unter dem Begriff Beladungskörperstoff versteht man somit alle Materialien oder Stoffe die wasserdurchlässig sind, und/oder diese speichern können. Die Beladungskörperstoffe erfüllen einen wichtigen Zweck. Der Kapillardruck der porösen Keramik ist so stark, dass z.B. unsere Zunge sofort an der porösen Keramik kleben bleibt. Das liegt an der sehr hohen Dichte der porösen Keramik. Wird die poröse Keramik mit Pinsel oder Flüssigkeiten beauftragt, wird diese sofort weggesaugt, aber die farbgebenden Komponenten, sind langsamer als die Lösungsflüssigkeit. So wirkt die poröse Keramik wie ein Filter, in dem sich die farbgebenden Komponenten dann irgendwo verstopft konzentrieren. Der Verteilungssteuerungsschritt kann diese auflösen und neu im Keramikblock verteilen. Dieses kann aber mehrere Tage in dem z.B. Silikongehäuse mit den entsprechenden Umgebungsparameter bedeuten. Dem zu Grunde wird je nach Porosität des Keramikrohlings ein Beladungskörperstoff ausgesucht, der flüssigkeitsbremsend wirkt. Somit kommt es zu wesentlich weniger ungewünschten Konzentrationsansammlungen. Die farbgebenden Komponenten können dann schneller vom Verteilungssteuerungsschritt eine gewünschte farbgebenden Verteilung erreichen. Des Weiteren lassen sich verschiedene farbgebende Komponenten in zugeordneten Volumen in Schichten aufbauen, das heißt 50ml Gesamtvolumen der vorhanden keramischen Porositäten können in jeweils 10ml verschiedene Beladungskörperstoffen eingebracht werden. Durch gleichen Kapillardruck können jetzt fünf verschiedene Farbkomponente Lösungen übereinander gelegt und gespeichert werden, ohne dass sich diese vermischen, was in einer Flüssigkeit ansonsten nahezu unmöglich ist. Durch die kapillardruckenthaltenden Beladungskörperstoffvorrichtung entsteht so eine Kapillarunterdruckbeladung, weil eine farbgebende Lösung den Druck an die andere weiter gibt und somit grundverschiedene und/oder verlaufende Farbübergänge entstehen. Die Farbpigmentlösung und/oder Beladungskörperstoff können farbgebenden Komponenten und/oder brandfesten Pigmente und/oder Oxide und/oder färbenden und fluoreszierenden Metalloxide und/oder organischen oder anorganischen Salze mit einer Farbpigmentlösung herbeiführen mit oder ohne Druck. Das bedeutet, dass die Beladungskörperstoffe den gewünschten Kapillardruck bei feuchtem und/oder nassem Untergrund aufnehmen. Die Beladungskörperstoffe dienen dann als Farblösungsmittelspeicher von Farbpigmentlösungen zur vollständigen Beladung aller Porositäten der porösen Keramik. Unter farbgebenden Komponenten der Farbpigmentlösungen versteht man alle farbgebenden und nicht farbgebenden Komponenten die zu gewünschten und spannungsfreien sinterbaren Keramikrohling führt.

Erfindungsgemäß findet der gewünschte Verteilungssteuerungsschritt der farbgebenden Konzentrationen organischer oder anorganischer Salze in einem steuerbaren Abdichtung- und/oder Isolierungsmittel z.B. einer Form statt. Vorzugsweise kann dies aus einer z.B. Silikonform hergestellt werden. Erstaunlicherweise wurde entdeckt, dass auch in einer porösen Keramik die sich in einem z.B. Silikongehäuse befindet ein Verteilungssteuerungsschritt stattfindet, der der Konvektion eines Fluiden in einem Gefäß ähnlich ist. Bei dem erfindungsgemäßen Verfahren können alle Konvektionen Anwendung finden, vor einer chemischen Konvektion. Bei einer Lösung auch solutale Konvektion, bei der in Verwendung stehenden Salzlösung, auch haline Konvektion, sowie auch von der thermohaline Konvektion, sowie Mahagoni-Konvektion und elektrischen Konvention. Die Konvektion ist durch die Stoffeigenschaften, Formkörper, beeinflussten Strömungen, Energie, Entropie, Stoffe und elektrische Ladungen werden ausgetauscht unter anderem durch Diffusion, Fasenübergänge, Trocknung, Sorption, Verdampfen, Erstarrungen, Dissoziation, lissoziation Reibungen. Des Weiteren kann eine Fläche als Katalysator wirken. Aus diesen Gründen ist die Konvektion auch schwer zu berechnen. Man hat durch viele hunderte Versuche und auch tausende Möglichkeiten eine farblich präzise wirkende Verteilungssteuerungsschritt durch die haline Konvektion und deren Einstellungen herausgefunden. Die Konvektion aus Gravitation und Dichtunterschieden wird weiterhin gesteuert durch die Menge organischer nichtorganischer Salze sowie Temperaturunterschiede elektrostatische Felder und um den umgebenden Luftfeuchtigkeitsgehalt. Sowie der Ausbildung der offenen oder abgedeckten Flächendes des porösen Keramikrohling. Der poröse Keramikrohling kann rund, kann Höhen 10-50 mm oder einen Durchmesser von 10-150 mm haben oder Hufeisenformen besitzen oder der vergrößerten Form eines gesamten Kiefers entsprechend. Sowie materialsparende Formen und Aussparungen besitzen.

Zum Transport des Fluid der steuerbaren Farbpigmentlösung wird erfindungsgemäß Wasser oder eine Mischung aus Wasser mit einem organischen, insbesondere einem polaren organischen Lösungsmittel verwendet. Die farbgebenden Komponenten und/oder brandfesten Pigmente und/oder Oxide und/oder färbenden und fluoreszierenden Metalloxide und/oder organischen oder anorganischen Salze enthalten, und/oder erfolgt durch eine wässrige Lösung oder Destillat auf Alkoholbasis. Organische Lösungsmittel sind zum Beispiel aliphatische Alkohole. Das Lösungsmittel und/oder Transportfluid kann gegebenenfalls Additive, wie zum Beispiel Stabilisatoren oder Elektrolyte, Komplexbildner, Dispergiermittel usw., beinhalten. Die Additive sind entweder im Beladungskörperstoff oder in Farbpigmentlösung enthalten. Unter einer Farbpigmentlösung versteht man auch alle Lösungen die in einer porösen Keramik in den Porositäten befindlich sind und sich durch offene und geschlossene Flächen am porösen Keramikrohling gesteuert verteilen.

Es ist außerdem zweckmäßig, dass in die Farbpigmentlösung ein Oxidationsmittel, Aluminiumnitrat insbesondere Wasserstoffperoxid und/oder Soidsalzen und/oder Zirconium (IV) Oxynitrate Hydrate, eingebracht wird. Ebenfalls ist es zweckmäßig, dass in die Farbpigmentlösung Öle und/oder Benzine eingebracht werden. Sowie das eine Strömungsreduzierung und Filter zwischen Beladungskörperstoffspeicher und z.B. Silikongehäuse zu legen. Der kapillare Körper ist dann Strömungsreduzierungsfilter und sorgt erstens dafür, das möglichst wenig Konzentrationsansammlung im den porösen Keramikrohling gelangen und sorgt zweitens somit für eine Kapillarunterdruckbeladung. Da die Auflagenflächen der porösen Keramik immer mit Zirkonstaub behaftet sind, was die Farbpigmentlösung verunreinigt, so das Beladungskörperstoffe und/oder kapillare Strömungsreduzierung und Filter und Beladungskörperstoffspeicher sind.

Bei einer möglichen erfindungsgemäßen Verfahrensvariante werden die Farbpigmentlösungen und/oder brandfesten Pigmente und/oder Oxide und/oder färbende und fluoreszierende Metalloxide und/oder organischen oder anorganischen Salze in/unter einer Vakuumatmosphäre ins Konvektionsgehäuse beladen. Das Transportieren der farbgebenden Komponenten und/oder brandfesten Pigmente und/oder Oxide und/oder färbenden und fluoreszierenden Metalloxide und/oder organischen oder anorganischen Salze der Farbpigmentlösungen kann auch unter einer annähernden Vakuumatmosphäre stattfinden. Dies ist jedoch nicht zwingend erforderlich. Zu starkes Vakuum kann je nach Porosität des keramischen Rohlings zu nicht gefühlten Porositäten und/oder zu ungewünschten Konzentrationsansammlungen der farbgebenden Komponenten führen.

Bei einer weiteren erfindungsgemäßen Verfahrensvariante werden die Farbpigmentlösungen und/oder brandfesten Pigmente und/oder Oxide und/oder färbenden und fluoreszierenden Metalloxide und/oder organischen oder anorganischen Salze der haline Konvektionen unter Druck transportiert. Somit kann auch der Kapillardruck durch Druckbeaufschlagung der kapillardruckenthaltenden Beladungskörperstofflösungsmittelvorrichtung erhöht werden. Zu viel Druck kann zur Über-und Unterschlagung von Porositäten und deren Hohlräumen führen und starke Verstopfungen verursachen und somit zu ungewünschten Konzentrationsansammlungen der farbgebenden Komponenten führen. Unter der kapillardruckenthaltenden Beladungskörperstofflösungsmittelvorrichtung versteht man alle dichten Auflagen oder Aufnahmevorrichtungen in den oder auf denen, sich Lösungen mit Farb- und nicht farbgebenden Komponenten unter Erhöhung und/oder Reduzierung des Kapillardrucks in den Porositäten der Keramik speichern lassen.

Bei dem erfindungsgemäßen Verfahren werden die Farbpigmentlösung mit und/oder brandfesten Pigmente und/oder Oxide und/oder färbenden und fluoreszierenden Metalloxide und/oder organischen oder anorganischen Salze in die Poren der porösen Keramik mit einem Beladungskörperstoffe unter kapillardruckhaltenden Farblösungsmitteln beladen und/oder Kapillarunterdruckbeladung eingebracht. An der Oberfläche der Keramik sind Porenöffnungen vorhanden, so dass die farbgebenden Komponenten und/oder brandfesten Pigmente und/oder Oxide und/oder färbenden und fluoreszierenden Metalloxide und/oder organischen oder anorganischen Salze in diese eindringen. Die Eindringtiefe ist abhängig von der Menge der Farbpigmentlösungsmittel und eingestelltem Kapillardruck, der kapillardruckenthaltende Beladungskörperstofflösungsmittelvorrichtung des Kapillardrucks. Der kapillardruckenthaltende Beladungskörperstofflösungsmittelvorrichtung sollte niedriger sein. Als der Kapillardruck der porösen Keramik und gleichmäßig und nicht zu schnell und ohne Lufteinschlüsse alle Porositäten mit Farbpigmentlösungen beladen werden.

Zweckmäßigerweise werden die Beladungskörperstoffe mit farbgebenden Komponenten gefüllt und als Beladungskörperstoffe lösungsmittelspeicher verwendet, die als einfaches Lager der Farbmischungen oder als Filter dienen. Wie z.B. Bierdeckel enthalten dann die gewünschten Konzentrationen und farbgebenden Komponenten der Zahnfarbe. Weiterhin bildet er Kapillarreduzierung der kapillardruckenthaltenden Beladungskörperstofflösungsmittel aus. Durch Auflegen der Bierdeckel auf die poröse Fläche des porösen keramischen Rohlings der sich in dem z.B. Silikongehäuse befindet. Das z.B. Silikongehäuse wird nun mit poröser Keramik auf die kapillardruckenthaltende Beladungskörperstofflösungsmittelvorrichtung flächig aufgelegt. So kann nun die Farbpigmentlösung an beliebige Stellen im porösen Keramikblock beladen werden. Unter Beladungskörperstoffen versteht man alle Materialien und Stoffe die flüssigkeitsdurchlässig sind und/oder strömungsreduzieren und/oder Kapillardruck halten können, in Bezug auf die poröse Keramik.

Das erfindungsgemäße Verfahren bietet außerdem die Möglichkeit, dass die gleichmäßige und/oder Konzentrationsverteilung der Verteilungsströmungsschritt wird durch das z.B. Silikongehäuse Nitratgehalt, Temperatur, Luftfeuchtigkeitsgehalt und mit verschiedenen großen Flächen die mit umgebender Luftfeuchtigkeit in Berührung stehen, gesteuert. Es ist dabei zu achten, dass die umgebende Luftfeuchtigkeit nicht unter ca. 30% sinkt, bei Temperaturen um ca. 25°. Da ansonsten die Bewegungsrichtung der Farbpigmente zu einer starken Konzentrationsansammlung führt, die nur selten gewünscht ist. Diese Beobachtung ist aber abhängig von der jeweiligen vorhandenen Porosität des Keramikkörpers.

Während des erfindungsgemäßen Verfahrens ist es vorteilhaft, wenn Farbmessungen an natürlichen Zähnen in einem programmierbaren Speicher hinterlegt werden, so dass die Farbe der natürlichen Zähne mit dem entsprechenden Farbpigmentlösungen reproduziert werden kann. Die Zahnfarbe der natürlichen Zähne des Patienten wird demnach gemessen. Anschließend werden die ermittelten Farbdaten in einer Software oder in einem programmierbaren Speicher gespeichert. Die Software oder der programmierbare Speicher kann mittels der Farbdaten die natürliche Zahnfarbe mit Farbpigmentlösungen berechnen bzw. reproduzieren. Der Farbverlauf wird dann graphisch vom CAD/CAM System angezeigt wird.

Des Weiteren wurde erstaunlicher Weise entdeckt, dass durch Abdeckungen bzw. Abkleben von längslaufende Zebrastreifen unter nicht abgeklebten Zebrastreifen der porösen Keramik im Keramikblock eine verlaufende räumliche Farbkonzentrationswelle oder bei offenen oder kreisrunden Abklebungen ein räumliche Farbkonzentrationskonuskegel entsteht. Wenn man die gewünschten Felder mit verschiedenen hohen Rahmen umgibt, stellt sich im Rahmen selbst eine höhere Luftfeuchtigkeit ein, obwohl eine konstante Luftfeuchtigkeit vorhanden ist. Die Flächen mit hohem Rahmen und höherer Luftfeuchtigkeit bilden eine vertikal verlaufende höhere räumliche Farbkonzentration. Flächen mit niedrigem Rahmen und niedriger Luftfeuchtigkeit bilden eine farbintensivere und niedrigere räumliche Farbkonzentration im porösen Keramikblock.

Eine weitere Möglichkeit des Verfahrens der Erfindung ist, dass eine programmgesteuerte Maschine oder Gerät, insbesondere eine CAD/CAM-Anlage, aus den zur Verfügung stehenden Konzentrationszonen Felder der Keramikrohlinge die Farbkonzentration heraussucht und heraus fräst, die der Zahnfarbe der natürlichen Zähne entsprechen.

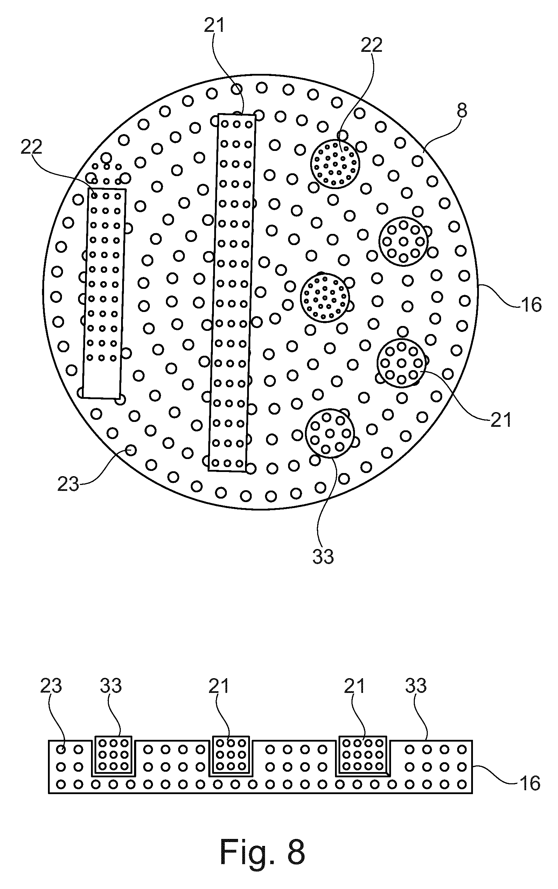

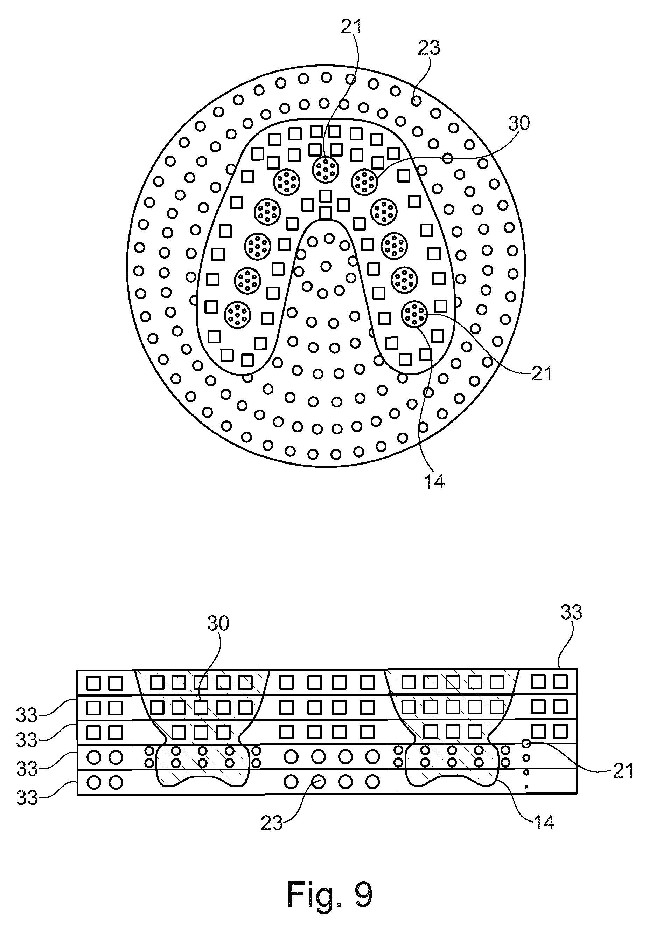

Weiterhin kann der Beladungskörperstoffe ausgefräst werden, zu einer gewünschten farbverlaufenden Figurgebung siehe Fig. 8 und Fig.9 oder die Anatomie der Oberfläche eines Zahnersatzes haben.

Es werden des Weiteren erfindungsgemäß die farbgebenden Farben gespeichert der durchgeführten Farbmessung an die programmgesteuerte Maschine oder an das programmgesteuerte Gerät übermittelt. Die zuvor ermittelten Farbdaten der natürlichen Zähne des Patienten werden an die programmgesteuerte Maschine oder Gerät übermittelt, so dass die Maschine einen oder mehrere Beladungskörperstoffe verwendet, und diese in ein z.B. Silikongehäuse einlegt, um die Zahnfarbe der natürlichen Zähne des Patienten herzustellen. Zur Einstellung der gewünschten Farben ist ein Spectrophotometer hilfreich. Hierbei ist L*-Wert Helligkeit(100 vollständige Reflektion, O: keine Reflektion)

$$a^* = \text{Wert Rot-Grünverfärbung}$$

(+a*: rot, -a: grün)

$$b^* = \text{Wert Gelb-Blauverfärbung}$$

(+b: gelb, -b: blau)

Die Nitratfarbstoffe z.B. 0,05 - 0,7 Gew.-% Chromoxid oder 0,05 - 0,2 Gew.-% Kupfernitrat oder Chromnitrat zu zugeben werden, erhält der Pressling eine braune oder grüne Farbe. Für eine rötlich rosa Farbe können 0,05 -2 Gew.-% Erbiumnitrat zugegeben werden. Für eine gelbe Farbe können 1-2 Gew.-% Cer-Nitrat oder 0,05-2 Gew.-% Vandiumnitrat zugegeben werden. Für eine violette Farbe können 0,5-2Gew.-% Neodymnitrat oder 0, 05-0,3 Gew.-% Cobaltnitrat zugegeben werden. Für eine orangegelbe Farbe können 0,05-0,5 Gew.-% Eisennitrat zugesetzt werden. Es kann eine Vielzahl von Farbstoffen auch gemeinsam verwendet werden, um die L* a* b* Werte einzustellen.

Die Nitratelement können jeweils in eine farbstabilisierende Vorauflösung eingebracht werden, aus 0,0001-60 Gew.-% Zirkonnitrate bezogen auf den Feststoff der Keramik.

Selbstverständlich muss die z.B. vorgesinterte Keramik eine zur Beladung offene Porosität aufweisen, damit in einem Verteilungsteuerungsschritt die farbgebenden Elemente, Parameter gesteuert in der Porosität bewegt werden können. Solche (offenen) Porositäten liegen bei >3% bis 90% besonders bevorzugt zwischen 25%-30%. Die in die farbstabilisierende Vorauflösung eingebrachten Nitrate und Nitratformeln beinhalten 0,0001-30% Gew.-% bevorzugt zwischen 0,0001 Gew.-% und 3 Gew.-% um z.B. eine Vita classical Farbescala von 16 Farben A1-D4 zu erreichen können z.B. folgende Kombinationen eingesetzt werden.

1. $Er(N_3)_3 \cdot 5H_2O + Fe(No_3) \cdot 9H_2O$
$Pr(No_3)_3\ 6H_2O +$

2. $Y(No_3)_3 \cdot 6\ H_2O + Er(No_3)_3 \cdot 5\ H_2O$
$FeNo_3) \cdot 9\ H_2O + Al(No_3) \cdot 9\ H_2O$

3. $Pr(No_3)_3 \cdot 6\ H_2O + Fe(No_3) \cdot 9\ H_2O +, Tb(No_3) \cdot 5\ H_2O + Mn(No_3)_2 \cdot 4\ H_2O$
oder
$Pr(No_3)\ 6\ H_2O + Tb(No_3) \cdot 5H_2O\ Fe(No_3)\ 9\ H_2O, Cr(No_3)_3 \cdot 9\ H_2O$
zu Feineinstellungen sind zusatzweise
$Yb(No_3)_3 \cdot 5\ H_2O\ Er(No_3), Dy(No_3)_3 \cdot X\ H_2O\ Eu(No_3)_3 \cdot 5\ H_2O, Nd(No_3)_3 \cdot 6H_2O,, V(No)_3 \cdot H_2O,, Ti(No_3)_4 \cdot 4H_2O$
zugeben

4. $Y(No_3)_3 \cdot 6H_2O, Er(No_3)_3 \cdot 5H_2O$
$Pr(No_3)_3 \cdot 6H_2O, Fe(No_3) \cdot 9H_2O, Zn(No_3)_2 \cdot 6H_2O$

5. $Fe(No_3)_3 \cdot 9\ H_2O, Cr(No_3)_3 \cdot 9\ H_2O, Ni(No_3)_2 \cdot 6\ H_2O, Mn(No_3)_2 \cdot 4\ H_2O$

6. $Fe(No_3)_3 \cdot 9\ H_2O, Cr(No_3)_3 \cdot 9\ H_2O, Mn(No_3) \cdot 4\ H_2O$

7. $Fe(No_3)_3 \cdot 9\ H_2O, Ni(No_3)_2 \cdot 6\ H_2O$

8. $Fe(No_3)_3 \cdot 9\ H_2O, Ni(No_3)_2 \cdot 6\ H_2O, Mn(No_3) \cdot 4\ H_2O$

9. Fe(No$_3$)$_3$ · 9 H$_2$O, Cr(No$_3$)$_3$· 9 H$_2$O

10. Er(No$_3$)$_3$ · 5H$_2$O, Fe(No$_3$)$_3$, Cr(No$_3$)$_3$ · 9 H$_2$O

11. Ni(No$_3$)$_2$ · 6H$_2$O, Pr(No$_3$)$_3$ · 6 H$_2$O

12. Fe(No$_3$)$_3$ · 9 H$_2$O

13. Er(No$_3$)$_3$ · 5 H$_2$O, Ni(No$_3$)$_2$ · 6 H$_2$O

14. Fe(No$_3$)$_3$ · 9 H$_2$O, Ni(No$_3$)$_2$ · 6 H$_2$O, Cr(No$_3$)$_3$ · 9 H$_2$O

15. Er(No$_3$)$_3$ · 5 H$_2$O, Fe(No$_3$)$_3$ · 9 H$_2$O

16. Fe(No$_3$)$_3$ · 5H$_2$O, Bi(No$_3$)$_3$ · 5 H$_2$O, Ce(No$_3$) · 6 H$_2$O

17. Er(No$_3$)$_3$ · 5H$_2$O, Pr(No$_3$)$_3$ · 6H$_2$O, Fe(No$_3$)$_3$ · 9H$_2$O, Zn(No$_3$)$_2$ · 6H$_2$O

18. Fe(No$_3$)$_2$ · 5H$_2$O, Cr(No$_3$)$_3$ · 9H$_2$O, Al(No$_3$)$_3$ · 9H$_2$O

19. Sr (No$_3$)$_2$, La(No$_3$)$_3$ · 6H$_2$O, Ag(No$_3$), Mg(No$_3$)$_2$ · 6H$_2$O, Fe(No$_3$)$_3$ · 9H$_2$O, Y(No$_3$)$_3$ · 6H$_2$O, Au Cl, Zn(No$_3$)$_2$ · 6H$_2$O, Eu (No$_3$)$_3$ · 5H$_2$O

20. Fe(No$_3$)$_3$ · 9H$_2$O, Al (No$_3$) · 9H$_2$O, Er(No$_3$)$_3$ · 5H$_2$O

21. Ce(No$_3$) · 6H$_2$O, Dy(No$_3$)$_3$ · xH$_2$O

22. Pr(No$_3$)$_3$ · 6H$_2$O, Fe(No$_3$)$_3$ · 9H$_2$O, Tb(No$_3$)$_3$ · 5H$_2$O

23. Ce(No$_3$)$_3$ · 6H$_2$O, Y(No$_3$)$_3$ · 6H$_2$O, Al(No$_3$)$_3$ · 5H$_2$O

24. Ce(No$_3$)$_3$ · 6H$_2$O, Y(No$_3$)$_3$ · 6H$_2$O, Al(No$_3$)$_3$, Mn(No$_3$)$_3$ · 4H$_2$O, Mg(No$_3$)$_2$ · 6H$_2$O

25. Ce(No$_3$)$_3$· 6H$_2$O, Fe(No$_3$)$_3$ · 9H$_2$O, Al(No$_3$) · 9H$_2$O, Co(No$_3$)$_2$ · 6H$_2$O

26. Fe(No$_3$)$_2$ · 9H$_2$O, Cr (No$_3$)$_3$ · 9H$_2$O, Cu (No$_3$)$_2$ · xH$_2$O, Y(No$_3$)$_3$ · 9H$_2$O, Pr(No$_3$)$_3$ · 6H$_2$O, Co(No$_3$)$_2$ · 6H$_2$O, Nr(No$_3$)$_2$ · 6H$_2$O, Mn(No$_3$)$_2$ · 4H$_2$O, Er(No$_3$)$_2$ · 5H$_2$O, Ce(No$_3$)$_3$ · 6H$_2$O

Die Charakterisierung der Farben erfolgt nach dem CIE-L*a*b-System der Kommission Internationale (CIE) Wien 1986. In dieser Systematik beträgt der Vita classical Farbring16 Farben folgenden Wertebereich der erreicht und einzustellen ist:

L*50 bis 76
a* -2,0 bis 10,0
b* 4,0 bis 30,0

Durch die Möglichkeit, Farben auch erfindungsgemäß zu konzentrieren können aber auch

L* 2 bis 50
a* -28,0 bis 35,0
b* -15,0 bis 31,0
gemessen werden.

Durch die Möglichkeit erfindungsgemäß transparente Oxidkeramiken und/oder Glaskeramiken zu beladen, sind auch folgende Werte zu erreichen von

L* 76 bis 98
a* - 4,2 bis 28,0

b*-1,60 bis 34,0

Erstaunlicherweise stellte sich heraus, dass durch die Vorauflösung z.B. mit Zirkonnitrate sich die Farbintensität auch steuern lässt. Das heißt zum Vergleich eine Probe

Dicke 1,5mm mal 60mm mal Breite 12mm optisch gleich zu Probe mit
Dicke 4,0mm mal 60mm mal 12mm Breite ist.
Bei Proben mit Pulveroxideinfärbung in der Schichttechnik gepresst
Dicke von 1,5mm mal 65mm mal 12mm Breite, optisch ein viel helleren Farbton zur Probe mit Dicke 4,0mm mal 60mm x 12mm Breite. Dies hat extreme Auswirkungen auf die Ästhetik.

Das heißt, dass die Kronengerüste Schichtstärke, ca. 0,5mm bis 1,5mm, viel heller ist, wie die der fehlenden Zähne, also das Brückenglied mit Schichtstärke von ca. 4-10mm. Der Zahntechniker kann diesen Farbunterschied nur mit erheblichem Arbeitsaufwand ausgleichen. Mit dem erfindungsgemäßen Verfahren lässt sich durch die Zugabe von den beschriebenen verschiedenen Vorauflösungskonzentrationen eine Farbgebung einstellen, die im gleichen Farbton z.B. 1,5mm und 4,0mm optisch gleich ist. Hierzu wurden jeweils 1,5mm und 4,0mm dicke Proben aus 3Y-TZP in einem Sinterprogramm mit 3° pro Minute auf 1450° für 120 Minuten gehalten und im Ofen abgekühlt auf 200°. Die Proben wurden mit einem SF 600* Data Color bei der Industrielackfabrik Walburg GmbH gemessen. Als Referenzunterlage wurde ein weiser Hintergrund ausgewählt mit folgenden Werten

L*94,94, a*3,87, *-12,85

Probenpärchen:

1. 3Y-TZP aus Oxid pulvertechnisch eingefärbte Proben

$$4mm = \ L^*\ 72{,}96 \quad a^*0{,}65 \quad b^*13{,}19$$

$$1{,}5mm = L^*\ 77{,}04 \quad a^*1{,}78 \quad b^*17{,}96$$

ergibt ein Differenzbemessungsbereich von 9,72

2. reines 3Y-TZP gleicher Hersteller mit gesteuerter farbgebender Konzentration einer 7gew.-% Nitratvorauflösung mit farbgebenden Nitraten

$$4mm = L^*78{,}80 \quad a^*\text{-}0{,}24 \quad b^*\ 17{,}80$$

$$1{,}5mm = L^*\ 78{,}32 \ a^*\text{-}0{,}82 \quad b^*18{,}90$$

ergibt ein Differenzbemessungsbereich von 2,20

3. Zur Sicherheit wurde eine weitere Probe aus 3Y-TZP hergestellt, farbgebender Konzentration einer 7gew.-% Nitratvorauflösung mit farbgebenden Nitraten im dunklen Farbton (bräunlich)

$$4mm = L^*49{,}90 \quad a^*3{,}41 \quad b^*9{,}37$$

$$1{,}5mm = L48{,}13 \ \ a^*3{,}58 \ \ b^*9{,}42$$

ergibt ein Differenzbemessungsbereich von 1,84

Auch hier waren die 1,5mm dicke Proben zum Unterschied 4mm dicken Proben optisch nicht zu unterscheiden. Eine errechnete Verbesserung der Farbkonstanten von ca. 70%.

Bei einer vorteilhaften Verfahrensvariante werden ganze vorgesinterte und/oder vorgepresste Keramikblöcke vor der CAD/CAM Bearbeitung eingefärbt. Das bedeutet, dass Keramikblöcke bereits in unterschiedlichen Grundfarben hergestellt werden können, damit nur noch die Z.B. und/oder der räumliche Farbkonzentrationskonuskegel in gewünschte Flächen mit einem Verteilungsgesteuertem Schritt entstehen kann oder um einen thermische Ausdehnung WAK der farbgebenden Oxiden auszugleichen.

Für eine Verfahrensausführungsform wird eine porös gebrannte Dentalverblendkeramik oder eine porös gepresste Glaskeramik als Keramik verwendet. Das Verfahren ist bei allen porösen Keramiken anwendbar. Unabhängig ob es sich dabei um eine gepresste, gebrannte, gebinderte, entbinderte und/oder angesinterte Keramik handelt. Die Keramik muss jedoch Poren aufweisen, so dass die farbgebenden Komponenten und/oder brandfesten Pigmente und/oder Oxide und/oder färbenden und fluoreszierenden Metalloxide und/oder organischen oder anorganischen Salze in mindestens einer Bewegungsrichtung der Farbpigmente zulassen.

Bei dem Verfahren wird beispielsweise eine poröse Keramik aus Zirkonoxid ($ZrO_2$), Yttriumoxid ($Y_2O_3$), Hafniumoxid ($HfO_7$), Aluminiumoxid ($Al_2O_3$) Phosphoroxid ($P_2O_3$, $P_2O_4$, $P_4O_{10}$), Titanoxid ($TiO_2$), Zinnoxid ($SnO$, $Sn_2O_3$, $SnO_2$), Boroxid (($BO)_x$, $(B_2O)_x$), Bortrioxid ($B_2O_3$), Fluor ($F_2$), Natriumoxid ($Na_2O$), Bariumoxid ($BaO$), Strontiumoxid ($SrO$), Strontiumperoxid ($SrO_2$), Magnesiumoxid ($MgO$), Zinkoxid ($ZnO$), Zinnoxid ($SnO$, $Sn_2O_3$, $SnO_2$), Calciumoxid ($CaO$), Titanoxid ($TiO_2$), Nioboxid ($NbO$, $NbO_2$, $Nb_2O_5$), Tantaloxid ($TaO$, $TaO_2$, $Ta_2O_5$), Lanthanoxid ($La_2O_3$), Siliciumdioxid ($SiO_2$), Leucit, Vanadium(V)-oxid ($V_2O_5$), dotierte Spinelle und/oder weitere Oxide sowie Mischungen hiervon umfasst, verwendet.

Des Weiteren kann bei dem erfindungsgemäßen Verfahren Feldspaltkeramik, Zirkonoxid- und/oder Leuzitverstärkte-Keramik, Lithiumsilikat-Gläser oder Lithiumsilikat-Glaskeramik oder Lithiumdisilikat-Glaskeramik, Silikatkeramik und/oder Oxidkeramik eingefärbt werden. Als einzufärbende Keramik für das Verfahren kann jede Oxidkeramik oder eine Keramik auf Basis von Oxidkeramik verwendet werden. Oxidkeramiken sind Hochleistungskeramiken, die härter, verschleißfester, wärmebeständiger und spröder als Hartmetalle sind. Sie bieten demnach die wichtigsten Eigenschaften für eine Implantatprothetik oder ein Implantat und/oder einen Zahnersatz. Des Weiteren kann auch eine Keramik, die auf Basis von Glaskeramik und/oder Glas besteht, verwendet werden.

Bei einem erfindungsgemäßen Verfahrensschritt wird die farbgebende Komponente in den porösen Keramikrohlingen unter ansteigender Vakuumatmosphäre oder in einem sauerstofffreien oder annähernd sauerstofffreien Raum gesintert, um die farbgebenden Komponenten und/oder brandfesten Pigmente und/oder Oxide und/oder färbenden und fluoreszierenden Metalloxide und/oder organischen oder anorganischen Salze in der Keramik zu fixieren. Das bedeutet, dass die farbgebenden Komponenten und/oder brandfesten Pigmente und/oder Oxide und/oder färbenden und fluoreszierenden Metalloxide und/oder organischen oder anorganischen Salze in den Poren der Keramik durch einen Sintervorgang fixiert werden, und/oder in ein Oxid verwandelt werden im Entsintervorgang.

Das erfindungsgemäße Verfahren umfasst folgende Schritte:

(1) a Bereitstellen einer durchgängig porösen und/oder vorgesinterten und/oder entbinderten und/oder gebinderten Keramik, insbesondere eines Keramikkörpers für die CAD/CAM Bearbeitung

(2) b Abdichtung und/oder Isolierung mittels einer Form des Keramikkörpers

(3) c Beladung der Porositäten des Keramikkörpers

(4) d Verteilungssteuerungsschritt der farbgebenden Elemente

(5) e Flüssigkeitsentzug bis zum Ende der Verteilungssteuerung in den abgedichteten und/oder isolierten Keramikkörper

(6) f Trocknen des keramischen Rohlings zur Kristallbildung und/oder Wärmebehandlung zur Oxidphasenbildung

(7) g CAD/CAM Bearbeitung

(8) h Endsintern und/oder Sintern zur Fixierung in Oxide

In der erfindungsgemäßen Farbpigmentlösung sind Konzentrationen der farbgebenden Komponenten und/oder brandfesten Pigmente und/oder Oxide und/oder färbenden und fluoreszierenden Metalloxide und/oder organischen oder anorganischen Salze zwischen 0,001 Gew.-% und 60 Gew.-% oder bevorzugt zwischen 0,0001 Gew.-% und 3 Gew.-

% enthalten. Je nachdem welche Farbe erreicht werden soll, sind entweder geringe oder große Mengen an farbgebenden Komponenten und/oder brandfesten Pigmenten und/oder Oxiden und/oder färbenden und fluoreszierenden Metalloxiden und/oder organischen oder anorganischen Salzen erforderlich. Demnach sind die Konzentrationen der farbgebenden Komponenten und/oder brandfesten Pigmente und/oder Oxide und/oder färbenden und fluoreszierenden Metalloxide und/oder organischen oder anorganischen Salze auch in den Beladungskörperstoffen um die gewünschte Farbe zu erzeugen und zu speichern.

Erfindungsgemäß beinhalten die Farbpigmentlösung und/oder der Beladungskörperlösungsmittelspeicher und/oder die Beladungskörperstoffe Konzentrationen aller farbgebenden und nicht farbgebenden Komponenten die in der Verteilungssteuerungsschritt bewegbar sind, einsetzbar.

Die farbgebende Lösung wird beim Verteilungssteuerungsschritt stabil gelöst, um zu verhindern, dass aus gemischten Elementen der Farbpigmentlösung bei dem Verteilungssteuerungsschritt einzelne Elemente aus der Farbpigmentlösung in eine Bewegungsrichtung zer- und/oder absetzten können. Dazu eignen sich Vorauflösungen aus Z.B. Zirconium (IV) Oxynitrate Hydrate.

Erfindungsgemäß beinhalten alle bekannten farbgebenden Komponenten organische und anorganische Salze, die anwendbar sind und beim Entsinterbrand sich in ein Oxid verwandeln.

Ein erfindungsgemäßes Ausführungsbeispiel stellt einen kapillardruckenthaltenden Beladungskörperstofflösungsmittelvorrichtung aus porösen oder schwammartigen Stoffe dar, der auch eine Beladungskörperstofflösungsmittelspeicher ist ,da die farbgebenden Komponenten und/oder brandfesten Pigmente und/oder Oxide und/oder färbenden und fluoreszierenden Metalloxide und/oder organischen oder anorganischen Salze enthalten sind da. Dem Fachmann sind dadurch alle weiteren Möglichkeiten bekannt. Siehe Nitratliste

Ein weiteres erfindungsgemäßes Ausführungsbeispiel stellt ein Beladungskörper dar, das alle Formen besitzen kann , wobei die farbgebenden Komponenten und/oder brandfesten Pigmente und/oder Oxide und/oder färbenden und fluoreszierenden Metalloxide und/oder organischen oder anorganischen Salze mindestens eines der Elemente Yttrium, Eisen, Titan, Selen, Silber, Indium, Gold, Chrom, Kupfer, Praseodym, Kobalt, Nickel, Mangan, Erbium, Cer oder Seltenerdmetalle sowie Mischungen hiervon beinhalten, die in den Beladungskörperstofflösungsmittel eingebrachten farbgebenden Komponenten und/oder nicht farbgebenden Komponenten. Die Beladungskörper können auch unter Kapillardruck der Farbpigmentlösung in einer luftdichten Verpackung gelagert werden.

Des Weiteren beträgt die Schichtstärke der Beladungskörper, die in und/oder auf der kapillardruckenthaltende Beladungskörperstofflösungsmittelvorrichtung und als Beladungskörperstofflösungsspeicher und Kapillarkraftreduzierung und Filter eingesetzt werden, eine Stärke zwischen 0,01 mm und 250 mm. Die Schichtstärke variiert zwischen dem oben genannten Bereich und ist aber auch abhängig vom Durchmesser der keramischen Körpern, von der Porosität und Konzentration der Farbpigmentlösung. Die Farbpigmentlösung kann auch in den Beladungskörpern getrocknet werden, um dann auf der Beladungskörperstofflösungsmittelspeicher und/oder kapillardruckenthaltende Beladungskörperstofflösungsmittelvorrichtung den Kapillardruck wieder aufzubauen, das zum Beladung der Farbpigmentlösung in die Porositäten führt.

Es ist ebenfalls zweckmäßig, dass die Farbpigmentlösung mit den darin beinhalteten Füllstoffen und farbgebenden Komponenten und/oder brandfesten Pigmenten und/oder Oxiden und/oder färbenden und fluoreszierenden Metalloxiden und/oder organischen oder anorganischen Salzen als Beladungskörperstoff gespeichert wird. Das die Beladungskörperstoffe verschiedene geometrische Formen aufweisen, die adaptierbar sind. Das bedeutet auch, dass Farbpigmentlösungen Beladungskörperstoffe den gleichen Kapillardruck aufweisen. Die Beladungskörperstoffe können jetzt nebeneinander, übereinander oder hintereinander auf den keramischen Rohlingen adaptiert werden, ohne dass sich die Farbpigmentlösung vermischen und das durch die Größe bzw. Volumen und Materialauswahl der Beladungskörperstoffe auch eine wunschgemäße Menge an farbgebenden Komponenten speicherbar sind.

Es werden nicht alle Materialien und/oder Komponenten und nicht farbgebenden Komponenten beziehungsweise Stoffe aufgeführt, die in dem erfindungsgemäßen Verfahren beziehungsweise in dem erfindungsgemäßen Verteilungssteuerungsschritten verwendet werden können. Dem Fachmann sollten jedoch durch die obigen Ausführungen alle Möglichkeiten bekannt sein.

Weitere Einzelheiten, Merkmale, Merkmalskombinationen und Wirkungen auf der Basis der Erfindung ergeben sich aus der nachfolgenden Beschreibung bevorzugter, beispielhafter Ausführungsformen der Erfindung sowie aus den Zeichnungen. Diese zeigen in:

Fig. 1    Ablaufschema des erfindungsgemäßen Verfahrens

Fig. 2    Querschnitt monochromen Keramikrohling im z.B Silikongehäuse

Fig. 3    Querschnitt polychrom Keramikrohling im z.B. Silikongehäuse

Fig. 4    Querschnitt polychrom Keramikrohling und räumlicher Farbkonzentrationswelle oder räumlicher Farbkonzentrationskonuskegel im Silikongehäuse

Fig. 5    Querschnitt polychrom und räumlicher Farbkonzentrationswelle und räumlicher Farbkonzentrationskegel mit programmierbarer CAD/CAM Bearbeitung

Fig. 6    z.B. Silikongehäuse Konzentrationsausgleich in mit z.B. mit mindesten einem Beladungskörper

Fig. 7    z.B. Silikongehäuse zum Konzentrationsausgleich der Farbkonzentrationen mit mindestens einem Beladungskörperlösungsmittelspeicher Querschnitt mit Beladungskörper

Fig. 8    Querschnitt Beladungskörper

Fig. 9    Querschnitt Beladungskörper gestapelt

Fig. 10   Prothesenkörper Querschnitt im Block

Fig. 11   Querschnitt Komplettsystem z.B. Silikongehäuse kapillardruckenthaltende Beladungskörperstofflösungsmittelvorrichtung

Fig. 12   Einen Querschnitt durch einen porösen, keramischen Körper, eine Verteilungssteuerungsvorrichtung sowie einen Beladungskörper.

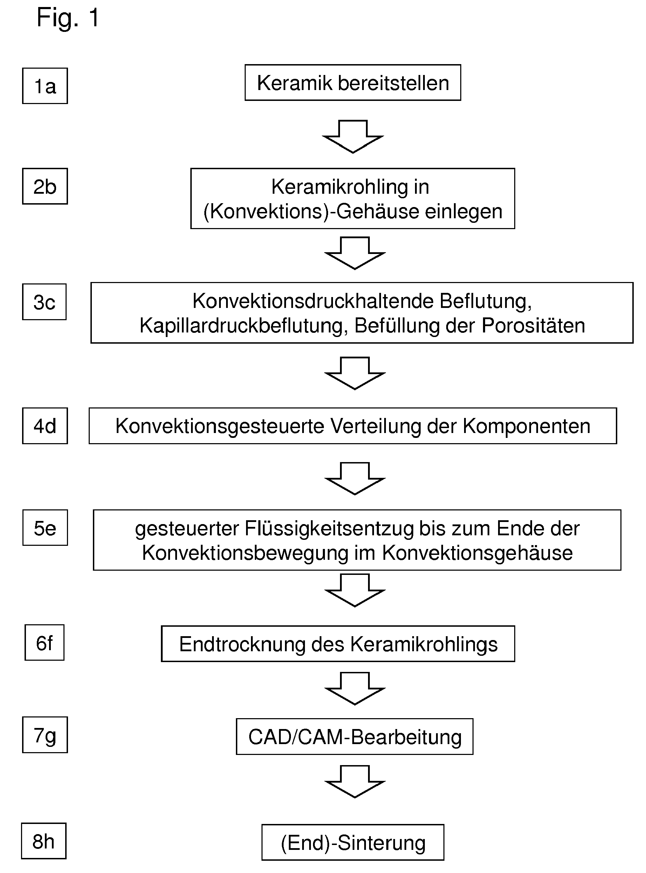

Die Fig.1 stellt ein Ablaufschema des Erfindungsgemäßen dar.

A) es werden nur poröse oder und/oder vorgesinterte und/oder entbinderte und/oder gebinderte Keramik für die CAD/CAM Bearbeitung bereitgestellt.

B) Der Keramikkörper wird dann in eine möglichst luftdichtschließende abdichtungsisolierende Form gelegt, z.B. in ein Silikonformgehäuse. Das z.B. Silikongehäuse kann in allen seinen Größen, allen Parametern frei wählbar sein und keinen atmosphärischen Druck haben und/oder einen solchen aufbauen können.

C) Anschließend wird durch luftfreien Beladung der kapillardruckenthaltende Beladungskörperstofflösungsmittelvorrichtung die Farbpigmentlösung in die Porositäten des porösen Keramikrohlings beladen. Hierzu eignen sich alle Beladungskörperstoffe zur auf oder in der kapillardruckenthaltende Beladungskörperstofflösungsmittelvorrichtung die auch einen kapillardruckgesteuerter Ausgleichstank besitzen können, um Kapillardruck konstant zu halten.

D) Im z.B. Silikongehäuse beginnt jetzt der Verteilungssteuerungsschritt der farbgebenden Komponenten in der porösen Keramik. Das z.B. Silikongehäuse hält oder bildet zudem offene und geschlossene Flächen aus, die mit einer bestimmten Luftfeuchtigkeit umgeben sind, um mit der Bewegungsrichtung der Farbpigmente gewünschte Farbgebungen zu erreichen.

E) Flüssigkeitsentzug bis Ende der Verteilungssteuerungsschritt

F) Trocknung der porösen Keramik und/oder Wärmebehandlung zur Nitratoxidphasenbildung

G) CAD/CAM Bearbeitung

H) Wärmebehandlung, Fixieren und Umwandlung der farbgebende Komponenten in Oxide zur Farbgebung, sowie die Entsinterung in einem Sinterbrand.

Das bedeutet, dass das z.B. Silikongehäuse mindestens an den Seitenflächen der fräsbaren Keramikrohlings möglichst dicht abschließt, da wie in einem Gefäß eine Konfektionsströmung eingestellt werden kann. In der farbgebenden Lösung eingebrachte farbgebende Komponenten können jetzt gleichmäßig oder im gewünschten Konzentrationsverlauf verteilt werden. Ohne Abdichtung und/oder Isolierung mittels einer Form ist dies aber nicht steuerbar möglich. Es kommt zu Stauungen oder zu nicht eingefärbten Bereichen, wie die EP 235 97 71 lehrt. Durch Vergrößerung und Verkleinerung z.B. Silikongehäuse an gewünscht offenen Flächen, lassen sich unter Temperatur und umgebender unterschiedlicher Luftfeuchtigkeit, mehrfarbige räumliche Farbkonzentrationswellen oder räumliche Farbkonzentrationskonuskegeln einfach im Keramikkörper festlegen, die dann den natürlichen Zahnnachbau bilden können. Z.B. die EP 29 19 771 und/oder Noritake lehren einen Keramikblock von dunkel nach hell zu schichten und Kronen mit viel Schneide aus der hellen Einstellungszone herauszufräsen. In der Praxis gibt es aber Kronen mit viel Schneide und intensiven Farbkern, der ist jetzt aber in den nur von dunkel nach hell geschichteten Block nicht möglich, weil in der Zone mit viel Schneide keine Dentinfarbe mehr geschichtet ist. Wolz lehrt einen beliebigen Winkel der vertikalen und horizontallen Zahnachse einstellbar und verschiebbar sind und/oder in der Konzentration der 3 räumliche Farbkonzentrationswelle oder dem räumliche Farbkonzentrationskonuskegel um 360° gedreht werden können. So sind erstmalig eine Vielzahl von ästhetischen Möglichkeiten die programmierbar, sowie Farbzonen die zur Verfügung stehen, die einen räumliche Farbverlauf haben.

Die Beladungskörperstoffe können unterschiedliche Konzentrationen nebeneinander besitzen oder können hintereinander gesteuert werden. Durch die kapillardruckenthaltende Beladungskörperstofflösungsmittelvorrichtung werden diese in gewünschte Positionen der Porosität des Keramikrohlings gebracht, die somit gesteuert vollständig die Porositäten belegen können. Es stellte sich heraus, dass bei gleichem Kapillardruck die Farbpigmentlösung hintereinander oder nebeneinander gehalten werden, ohne sich zu vermischen. Somit ist auch eine Mehrfarbigkeit erreichbar, die durch einen regelbaren Verteilungssteuerungsschritt der Farbpigmente erstmalig z.B. in einem Silikongehäuse wunschgemäß verteilt werden.

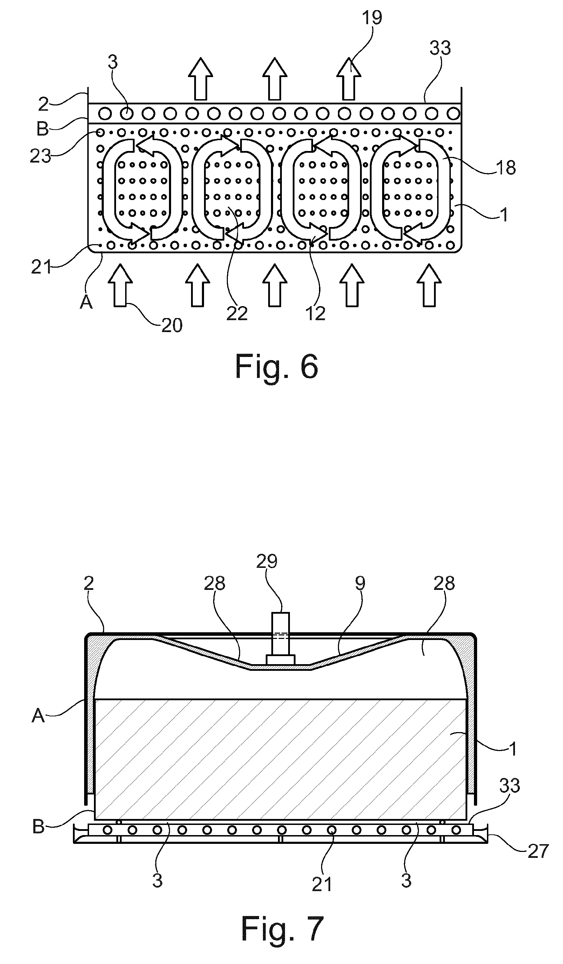

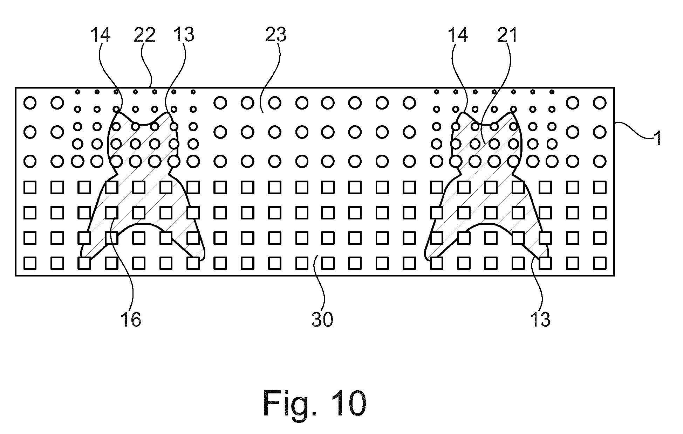

Erfindungsgemäß befindet sich am oder unter dem z.B. Silikongehäuse ein Beladungskörperstofflösungsmittelspeicher, der mit porösen und/oder schwammartigen Stoffen wie z.B. Mikrofaser, Schwämme, Zellstoff usw. gefüllt ist. Der Beladungskörperstofflösungsmittelspeicher sollte doppelt so viel Farbpigmentlösungen im Volumen aufnehmen können, wie die bereitgestellte poröse Keramik. Die Beladungskörperstoffe speichern auch farbgebende und nichtfarbgebenden Komponenten, die zu einem Spannungsausgleich von sehr hohem Farbkonzentrationen in nur einem Bereich des keramischen Rohlings beim Entsinterbrand führen, die dann auf der gegenüberliegenden Seite ausgeglichen werden z.B. Fig. 6. Des weiteren ist die Herstellung einer Teil- und/oder Totalprothesenrohlings der eine Zahnfarbe, Zahnbogen und einen rosafarbenen Anteil besitzt, die mit der Verteilungssteuerungsschritt regelbar in der z.B. Silikongehäuse gewünschte Farbgebung erreicht, siehe Fig. 10.

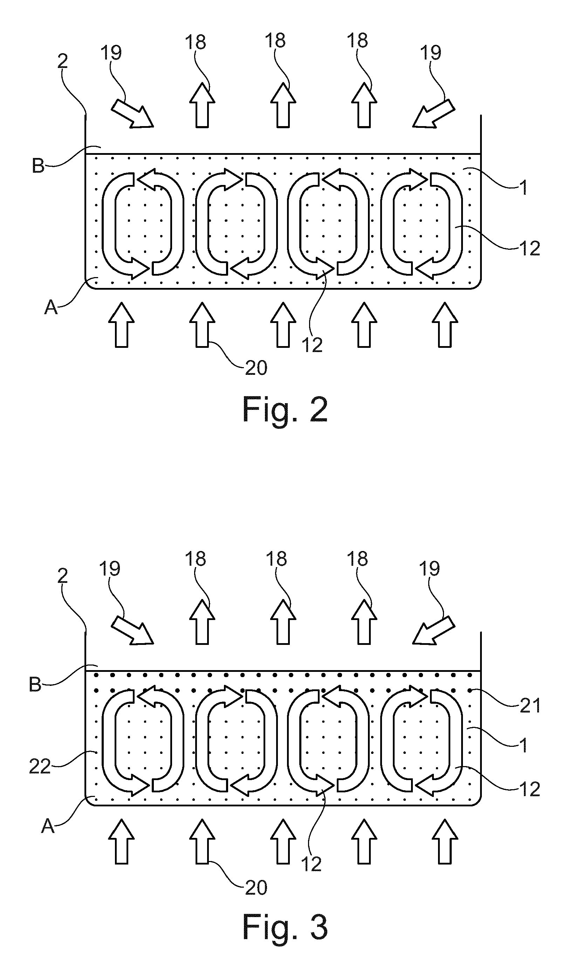

Fig. 2 stellt den Querschnitt eines monochromen porösen Keramikrohlings dar, der mit der kapillardruckenthaltende Beladungskörperstofflösungsmittelvorrichtung beladen wurde. Im z.B. Silikongehäuse wird Flüssigkeit entzogen. Der poröse Keramikrohling ist während der Verteilungssteuerungsschritt der gewünschten Konzentration in dem z.B. Silikongehäuse umgeben. Der poröse Keramikrohling stellt ohne Silikongehäuse einen keramischen Filter dar, bei dem sich bei der Infiltration Konzentrationen ansammelt die nicht gewünscht sind, wie die Lehre der DE 10 2008 026 980 lehrt. In einem z.B. Silikongehäuse lässt sich erstaunlicher Weise aus diesen Konzentrationsansammlungen in einem porösen Keramikrohling eine Bewegungsrichtung der Farbpigmente verteilend steuernd bewegen. Mit angegebenen komplexen Beladungsmittellösungen können sowohl räumliche Polychrome, oder monochrome Einfärbungen in vorgefertigte poröse keramische Rohlinge beladen werden. Die verschiedenen Farben und Chromatas sind dem keramischen Fachmann bekannt und auch in den aufgeführten Schriften beschrieben. Z.B. eine 7% Zirkonnitratlösung wird angesetzt um chemisch stabile Auflösung zu erhalten. Diese Lösung wird auf dem Rollenbock mit ca. 20 Umdrehungen pro Minute 24 Stunden gemischt. Danach erfolgt die Ermittlung des Porositätsvolumens des gewünschten vorgefertigten keramischen Rohlings. Bei der in der Verwendung stehenden DD Bio Zirkon der Fa. Dental Direkt Zx2 Durchmesser 98mm Höhe 14mm 3 Yittrium TZP Charge Nr. 50143002 Gewicht 330gr, war eine Beladungsvolumen der Porositäten von 50gr Beladungsmittel möglich. Dies ist je nach Hersteller neu zu ermitteln. Nach der Ermittlung können jetzt verschiedene Pigmente der farbgebenden Salze zugegeben werden. Auf 50gr vorgelöster Beladungsmittellösung werden 1-6gr Erbium und 0,1 bis 1gr Eisen zugemischt und 1-24 Stunden auf dem Rollenbock gemischt. Die Farbeinstellungen sind abhängig von der Porosität, Reinheit der Grundmaterialien und gewünschten Farbverlauf des Chromata. Bei der Herstellung von monochromen keramischen Rohlingen kann man folgender Maßen vorzugehen. Die farbgebenden Komponenten werden auf Fläche A des porösen Keramischen Rohlings, durch kapillardruckenthaltende Beladungskörperstofflösungsmittelvorrichtung, eine kapillarunterdruck Beladung durchgeführt in ca. 25 min sind 50gr Beladungsmittel in die poröse Keramik eingebracht, dann um 180° gedreht, wenn das z.B. Silikongehäuse keine verstellbaren Abdeckungen hat siehe Fig. 2. Alle Farbkomponenten 4 wandern zur Fläche B des porösen keramischen Rohlings nach ca. 80%-90% Flüssigkeitsentzug unter 70% Luftfeuchtigkeit an Fläche B wird nun wieder um 180° gedreht, wenn das Konvektionsgehäuse keine verstellbaren Abdeckungen hat. Nach Vollständiger Trocknung und/oder Temperatureinwirkung zur Nitratoxidphasenbildung findet die Fräsbearbeitung statt. Somit findet eine homogene Verteilung der Konzentration statt, da die Flüssigkeit entzogen wird, bevor sich wieder Farbkonzentrationen bilden können. Die Angaben sind abhängig von eingestellter Viskosität der Farbpigmentlösung und Porosität des keramischen Rohlings. Die Wärmebehandlung für den gesinterten Keramikgegenstand , ist typischer Weise unter folgenden Bedingungen durchzuführen. Je nach verwendeten Keramiken sind Temperaturen von ca. 700°-1600° atmosphärisch: Luft oder Vakuum oder Inertgas (Stickstoff, Argongas), Druck: Umgebungsdruck, Dauer: bis eine Dichte von ca. 94% bis ca.100% der endgültigen Dichte des Material erreicht ist.

Fig. 3 stellt den Querschnitt eines polychromen porösen Keramikrohling dar bei dem ein farbgebender Konzentrationsverlauf von dunkel nach hell hergestellt wird. Fläche B wird mit kapillardruckenthaltende Beladungskörperstofflösungsmittelvorrichtung und im z.B. Silikonform Flüssigkeit entzogen. Die farbgebenden Komponenten können unter Kapillardruck stehen, in den Beladungskörperstoffe hintereinander, übereinander oder nacheinander eingebracht werden, je nach Anspruch des Farbwunsches. Eine einfache Kapillarunterdruckbeladung mit einer farbkompetenten Lösung erzeugt natürlich auch einen verteilungsgesteuerten Farbverlauf von dunkel nach hell der Fläche B, die mit einer Luftfeuchtigkeit von 30%-80% in Berührung steht. Man hat herausgefunden, dass z.B. 50% Luftfeuchtigkeit eine stärkere Bewegungsrichtung der Farbpigmente im Verlauf entsteht. Also mehr dunkel und mehr hell und bei 80% 3 Luftfeuchtigkeit eine leichte d.h. weniger dunkel weniger hell Bewegungsrichtung der Farbpigmente von dunkel nach hell. Der poröse Keramikrohling ist bis zur nicht mehr Bewegbarkeit die Farbkomponenten Flüssigkeiten zu entziehen. Die Trockenzeit ist abhängig von Porosität und Größe des porösen keramischen Rohlings und der Luftfeuchtigkeit, Raumtemperatur und damit verbundenen gewünschten Farbverlauf.

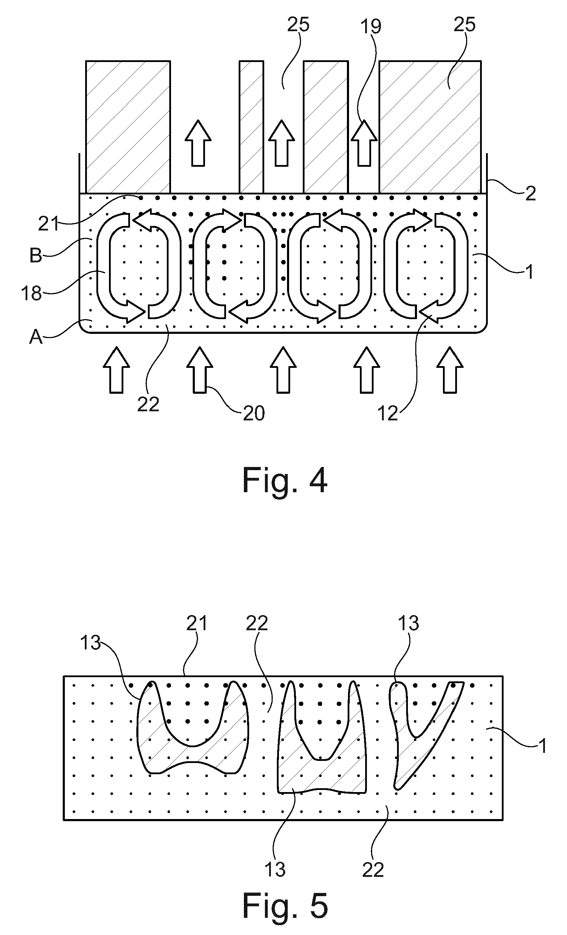

Fig. 4 stellt den Querschnitt eines polychromen Keramikrohlings dar, der dazu noch dreidimensionalen Farbzonen aufweist. Fläche B wird mit kapillardruckenthaltende Beladungskörperstofflösungsmittelvorrichtung und in Konvektionsgehäuse mit entsprechenden möglichst luftdichten Flächen und/oder Rahmen auf Fläche B gedrückt und Flüssigkeit entzogen. Zur Unterstützung von kapillardruckenthaltende Beladungskörperstofflösungsmittelvorrichtung können auch Beladungskörperstoffe Hilfsstoffe wie Fig.8 und Fig.9 als Beladungskörperlösungsmittelspeicher sein, die kapillardruckenthaltende Beladungskörperstofflösungsmittelvorricung unterstützen. Erstaunlicher Weise ist die ersichtliche Bewegungsströmung so stark, das auch eine einfache kapillardruckenthaltende Beladungskörperstofflösungsmittelvorrichtung mit einer farbgebenden Komponenten Lösung des porösen Keramikrohlings, der nach der Beladung z.B. mit Tesastreifen abgeklebt, um Öffnungen oder abdeckende Flächen zu erzeugen. Der Verteilungssteuerungsschritt kann so stark sein, dass aus allen Bereichen die mit farbgebenden Komponenten in den porösen Keramiken gefüllt sind, diese zu den offenen Flächen wandern.

Fig.5 Stellt einen Querschnitt eines porösen Keramikrohlings dar, der dazu noch dreidimensionale Farbzonen aufweist und getrocknet ist. Der Farbverlauf der Zonen wird graphisch auf einer Software dargestellt (13). Der Zahntechniker ganz selbst oder an Hand von digitaler Farbarten den gewünschten Zahnfarbenkonzentrationsverlauf (21 (22) aussuchen. Die CAD/CAM Anlage fräst dann aus gewünschten Zonen des keramischen Rohlings(1) den Zahnersatz(13) heraus.

Fig.6 Stellt einen Querschnitt eines porösen Keramikrohlings dar, der schon getrocknet ist und dereine starke Farbkonzentrationskomponente (21) im Flächenbereich A aufweist. Bei der Endsinterung je nach Porosität und Herstellungsverfahren der keramischen Rohlinge (1) kann es zu Spannungen innerhalb des endgesinterten keramischen Körpers kommen. Beim an sich bekannten Endsintern wird die poröse Keramik bzw. der keramische Rohling einer Hochtemperaturbehandlung ausgesetzt, wodurch eine Werkstoffverdichtung erzielt wird und die Porenräume aufgefüllt werden. Die genannten Spannungen können aufgrund unterschiedlicher Wärmeausdehnungskoeffizienten (WAK) der verschiedenen Materialien, bspw. des Keramikrohlings sowie der infiltrierten Farbpigmente, die zu einer abweichenden wärmebedingten Volumenausdehnung führen, entstehen. Einer Entstehung solcher Spannungen kann bereits im Herstellungsverfahren des porösen keramischen Rohlings vorgebeugt werden. Gibt man einfach, wie in Fig. 6, auf einen schon getrockneten porösen keramischen Rohling einen Beladungskörperstoff mit dem entsprechenden nicht farbgebenden Komponenten mit einer Kapillarunterdruckbeladung dazu. Es wird die Konzentration berechnet und mit nicht farbgebenden Komponenten (23) zu wie z.B. Zirconium (IV) Oxynitrate Hydrate und oder Zirkonverbindung II, III, IV und/oder mineralische organische Zirkonverbindung ausgeglichen. Siehe Tabelle 1. So befinden sich in den Porositäten der vorgefertigten Keramik z.B. verlaufende Nitratkomplexe Bereich(Schicht) mit z.B. 75%, 50%, 25% von färbenden Nitrate und zu gleichen Anteilen ausgleichende nicht farbgebende Nitrate.

Tabelle

3Y-TZP 12mm Dicke erfolgen

| Farbnitrate | | | Ausgleich mit nicht farbgebenden Nitrate |
|---|---|---|---|
| Verlaufende Nitrat komplex Schicht ca. 3mm | 75% | 25% | |
| Verlaufende Nitrat komplex Schicht ca. 3mm | 50% | 50% | |
| Verlaufende Nitrat komplex Schicht ca. 3mm | 25% | 75% | |
| Verlaufende Nitrat komplex Schicht ca. 3mm | 0% | 100% | |

Fig.7 Stellt einen Querschnitt der kapillardruckenthaltende Beladungskörperstofflösungsmittelvorrichtung in einer z.B. Silikonformgehäuse dar. Der poröse keramische Rohling wird in die z.B. Silikonformgehäuse gedrückt, mit Ventil oder Stempel (29) atmosphärischer Druck verringert. Der poröse keramische Rohling wird auf Fläche B auf trockene und/oder unter Kapillardruck stehende Beladungskörperstoffe der ein Beladungskörperlösungsmittelspeicher ist, eine Kapillarunterdruckbeladung erzeugt. Die Kapillarräume eines 14mm hohen und 98mm hohen Durchmesser eines Keramikrohlings benötigen ca. 50gr an Farbpigmentlösung zur Kapillarraumbefüllung, die durch eine Kapillarunterdruckbeladung im z.B. Silikongehäuse in ca. 25 min erreicht wird. Die Zeit ist aber abhängig von Farbpigmentlösung Konzentrierung der eingestellten Viskosität der Kapillardruck Unterstützung durch Verhältnisse in der Silikongehäuse und der Art des Materials und Größe der Porositäten.

Fig.8 stellt einen Querschnitt von Beladungskörperstoffen dar, die farbgebende oder nicht farbgebende Komponente in einer Kapillarunterdruckbeladung in der porösen Keramik erzeugen, dabei stehen die Beladungskörperstoffe unter gleichem Kapillardruck. So kommt es zu keiner Vermischung unterschiedlichen Farbkomponenten (21) oder nicht farbgebenden Komponenten (23). Somit können nebeneinander oder hintereinander oder übereinander alle möglichen Farbgebungen und Spannungsausgleiche durch Farbpigmentlösung in die poröse Keramik beladen werden. Ganz wichtig ist auch das jegliche farbgebende Form Konturen wie z.B. Kieferform, einzelne Zahnformen, Implantate, Abbumentformen, in horizontal oder vertikalem Querschnitt aus Beladungskörperstoffen ausgeschnitten, gefräst, gestanzt oder geplottet usw. hergestellt werden können. Sowie das Beladungskörperstoffe Kapillarvolumen des Konzentrationsverlaufes auf das Kapillarvolumen der porösen Keramik berechenbar sind.

Fig.9 stellt den Querschnitt von fünf Beladungskörperstoffen geschichtet dar, um dreidimensionale Prothesenkörper herzustellen. Z.B. als werden fünf Beladungskörperstoffe übereinander gelegt. Dazu eignen sich 1,4mm starke Bierdeckel im Durchmesser 104mm diese speichern ohne Probleme über 10g an Farbpigmentlösung und/oder farbgebender Komponenten. Das bedeutet, dass die benötigte Farbpigmentlösung Volumen in fünf Beladungskörperstoffe ohne Vermischung in die porösen keramischen Rohlinge unter Kapillarunterdruckbeladung auf der kapillardruckenthaltende Beladungskörperstofflösungsmittelvorrichtung durchgeführt. In dem z.B. Silikongehäuse wird nun die Flüssigkeit entzogen. Die Flüssigkeitsentzugsgeschwindigkeit pro 1,0mm der porösen Keramik beträgt dann 24 Stunden, je nachdem wie die Farbpigmentlösung zusammengesetzt und hergestellt wurde und/oder die Umgebung der Luftfeuchtigkeit von 50%-90% eingestellt ist.

Fig.10 stellt einen Querschnitt eines getrockneten porösen keramischen Rohlings dar, der mit polychromen räumlichen Farbverlauf aufweist. Der Farbverlauf des gesamten Prothesenkörpers wir graphisch von der Software dargestellt, Der Zahntechniker bestimmt selbst oder auf Grund digitaler Farbdaten den gewünschten Prothesenkörper Zahnfarbenverlauf. Die CAD/CAM Anlage fräst dann die gewünschten Prothesenkörper mit entsprechenden Farbverlauf aus dem porösen keramischen Rohling heraus.

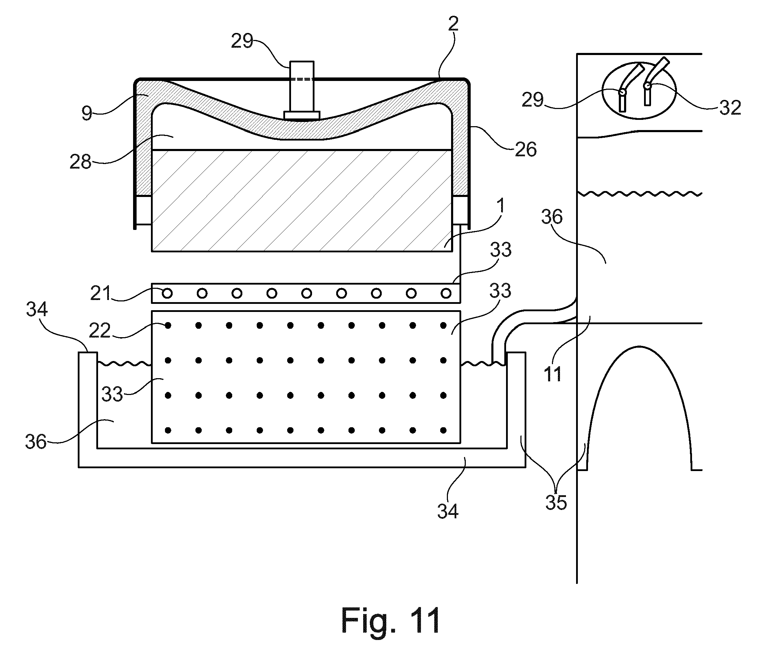

Fig. 11 stellt den Querschnitt des kompletten Systems dar, mit z.B. Silikonformgehäuse und porösen Keramikrohlingen (1) austauschbaren oder schichtbaren Beladungskörperstoffe (7) mit möglichen Farbkomponenten unter Kapillardruck(21). Kapillardruckenthaltende Beladungskörperstofflösungsmittelvorrichtung und Speicher aus (7) ( porösen oder schaumartigen Stoffen) mit und/oder ohne farbgebenden Komponenten als Speicher mit möglichen kapillardruckgesteuerter Ausgleichstank, mit unter atmosphärischen Druckventil und/oder Unterdruckaufschlagungsventil (29).

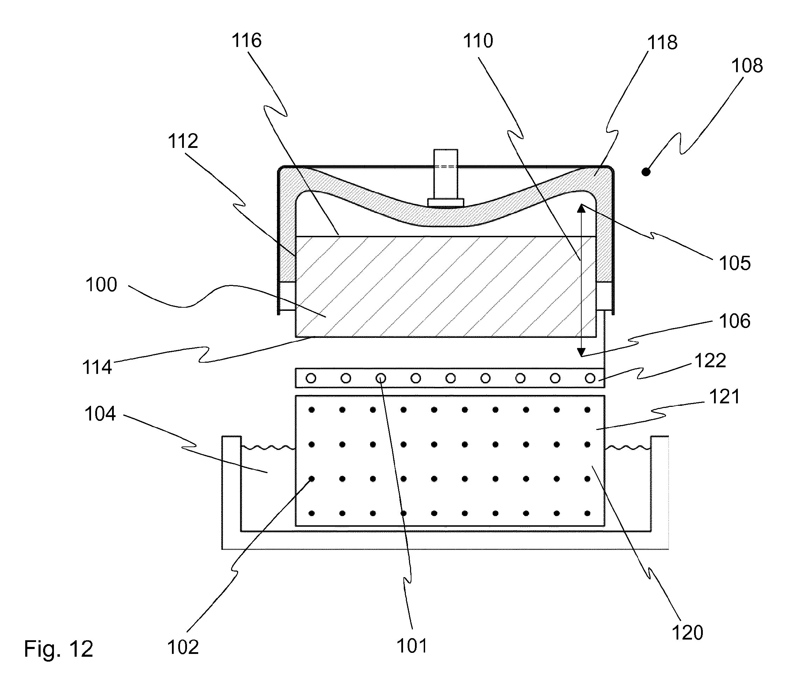

Gemäß Figur 12 ist der poröse keramische Körper 100 in eine Form 118, insbesondere eine Silikonform formschlüssig eingepasst, wobei eine frei zugängliche Oberfläche 114 zur Beladung mit Farbpigmenten 101, 102, die in einer Farbpigmentlösung 104 enthalten sind, auf einen Beladungskörper 120 mit zwei Schichten 121, 122 auflegbar ist. Bezüglich der frei zugänglichen Oberfläche 114 und einer isolierten und/oder abgedichteten Oberfläche 116 ist ein Umgebungsparametergradient 110 zwischen einem Parameter 105 innerhalb der Form 118 und einem Umgebungsparameter 106 in einer Umgebung 108 durch Regelung des Umgebungsparameters 106 und/oder Regelung des Parameters 105 einstellbar.

**Bezugszeichenliste**

1 - poröse und/oder gesinterte und/oder entbinderte und/oder gebinderte Keramik bereitstellen
2 - z.B. Silikonform mit eingelegtem porösen Keramikrohling
3 - Beladung der Porositäten des Keramikrohlings
4 - Verteilungsschritt der Komponenten
5 - Flüssigkeitsentzug bis Ende des Verteilungssteuerungsschrittes in z.B. Silikonform
6 - Trocknung des porösen Keramikrohlings
7 - CAD/CAM Bearbeitung
8 - Innenfläche des Zahnersatzes und/oder des Implantats oder Implantatprothetik
9 - Silikon
10- Ventil
11 -
12 - Zeichen für Verteilungssteuerungsschritt
13- gestrichelte Bilder zur geplanten CAD/CAM Bearbeitung
14- Prothesenzahn

15-rosafarbener Farbanteil

16-mögliche Einlagerung in 3 Dimensionale Farbraum

17-Farbkmponenten

18-Flüssigkeitentzug

19-Luftfeuchtigkeit

20-Tempertur

21-farbgebende Komponenten Konzentrationen (groß)

22- farbgebende Komponenten Konzentrationen (klein)

23-farblose Komponenten z.B. zum Konzentrationsausgleich groß

24- farblose Komponenten z.B. zum Konzentrationsausgleich klein

25-z.B. Silikongehäuseabdeckrahmen

26-

27-Gefäß

28-unter atmosphärischen Druckbereich oder ohne atmosphärischen Druck

29-unter atmosphärischen Druck auslösendes Ventil

30-Zahnfarbe rosa Zahnfleischfarbe

31-kappilardruck gesteuerter Ausgleichtank

32-Druckeinstellventile

33-Beladungskörper

34-kapillardruckenthaltende Beladungskörperstofflösungsmittelvorrichtung

35-Beladungskörperstofflösungsmittespeicher

36-Farbpigmentlösung

37-Stabilisatoren

a,b,c,d Einfärbung mit Abstufungen von hell bis dunkel mit und ohne Farbverlauf

100-poröse Keram i k

101, 102-Farbpigment

104-Farbpigmentlösung

105-Parameter

106-Umgebungsparameter

108-Umgebung

110-Umgebungsparametergradient

112-isolierter und/oder abgedichteter Teil einer Oberfläche

114-zweite, frei zugängliche Oberfläche

116-erste, isolierte und/oder abgedichtete Oberfläche

118-Form

120-Beladungskörper

121, 122-Schicht

## Patentansprüche

1. Verfahren zur Herstellung eines polychrom und/oder räumlich polychrom oder eines monochrom gefärbten keramischen Körpers, insbesondere eines derart gefärbten Dentalkeramikrohlings, **dadurch gekennzeichnet, dass** zur Steuerung einer gezielten Verteilung von Farbpigmenten (101, 102) innerhalb einer porösen Keramik (100), in einem ersten Schritt, der ein Beladungsschritt (3c) ist, die Keramik (100) mit einer Farbpigmentlösung (104) beladen wird, und in einem zweiten Schritt, der ein Verteilungssteuerungsschritt (4d) ist, die Verteilung der Farbpigmente (101, 102) innerhalb der Keramik (100) durch Regelung eines oder mehrerer Umgebungsparameter (106) in einer Umgebung (108), insbesondere durch Regelung der Luftfeuchtigkeit und/oder des Drucks und/oder der Temperatur, gesteuert wird.

2. Verfahren nach Anspruch 1, **dadurch gekennzeichnet, dass** die Verteilung der Farbpigmente (101, 102) innerhalb der porösen Keramik (100) durch eine Konvektionsströmung bewirkt wird, wobei Strömungsrichtung und - geschwindigkeit durch gezieltes Erzeugen von Umgebungsparametergradienten (110), insbesondere durch Einstellen von Luftfeuchtigkeitsunterschieden und/oder Druckunterschieden und/oder Temperaturunterschieden, bezüglich verschiedener Oberflächen (114, 116) der porösen Keramik (100) gesteuert werden.

3. Verfahren nach einem der vorhergehenden Ansprüche, **dadurch gekennzeichnet, dass** eine Bewegungsgeschwindigkeit der Farbpigmente (101, 102) und/oder die Strömungsgeschwindigkeit durch Erhöhen und/oder Verringern eines oder mehrerer Umgebungsparametergradienten (110) gesteuert wird.

4.  Verfahren nach einem der vorhergehenden Ansprüche, **dadurch gekennzeichnet, dass** eine Bewegungsrichtung der Farbpigmente (101, 102) und/oder die Strömungsrichtung durch Änderung der Richtung eines oder mehrerer Umgebungsparametergradienten (110) gesteuert wird.

5.  Verfahren nach einem der vorhergehenden Ansprüche, **dadurch gekennzeichnet, dass** mindestens eine Oberfläche (116) oder mindestens ein Teil einer Oberfläche (112) der porösen Keramik (100) während des Beladungsschritts und/oder während des Verteilungssteuerungsschritts isoliert und/oder abgedichtet ist und, dass mindestens eine andere Oberfläche (114) oder mindestens ein anderer Teil einer Oberfläche der porösen Keramik (100) zur Beladung und/oder zur Steuerung frei zugänglich ist.

6.  Verfahren nach einem der vorhergehenden Ansprüche, **dadurch gekennzeichnet, dass** das Lösungsmittel Wasser und Zirkon-Nitrat enthält.

7.  Verfahren nach einem der vorhergehenden Ansprüche, **dadurch gekennzeichnet, dass** eine Beladung der porösen Keramik (100) mit Farbpigmenten (101, 102) mittels eines Beladungskörpers (120), insbesondere aus einem porösen und/oder schwammartigen Material erfolgt, wobei der Beladungskörper mit einem Lösungsmittel und darin enthaltenen Farbpigmenten (101, 102) versetzt, insbesondere gesättigt ist.

8.  Verfahren nach Anspruch 7, **dadurch gekennzeichnet, dass** die poröse Keramik (100) zur Beladung mit Farbpigmenten (101, 102) mit einer frei zugänglichen Oberfläche (114) auf den Beladungskörper (120) aufgelegt wird.

9.  Verfahren nach Anspruch 7 oder 8, **dadurch gekennzeichnet, dass** der Beladungskörper (120) eine oder mehrere Schichten (121, 122) umfasst, wobei eine oder mehrere Schichten (121, 122) gleiche Farbpigmente (101, 102) zur Erzeugung einer monochromen Keramik oder verschiedene Schichten (101, 102) verschiedene Farbpigmente (101, 102) zur Erzeugung einer polychromen Keramik enthalten.

10.  Verfahren nach einem der vorhergehenden Ansprüche, **dadurch gekennzeichnet, dass** die poröse Keramik (100) oder der Keramikrohling nach der Verteilung der Farbpigmente (101, 102) in einem zusätzlichen Trocknungsschritt wärmebehandelt wird, wobei die poröse Keramik (100) oder der Keramikrohling zur Ausbildung einer Oxidphase einer Temperatur in einem Bereich zwischen 80°C und 1200°C, insbesondere zwischen 80°C und 800°C ausgesetzt wird.

11.  Verfahren nach einem der vorhergehenden Ansprüche, **dadurch gekennzeichnet, dass** während und/oder nach der Verteilung der Farbpigmente (101, 102) innerhalb der porösen Keramik (100) ein WAK-Ausgleich durchgeführt wird, wobei die poröse Keramik (100) zumindest teilweise mit einem Ausgleichsstoff beladen wird.

12.  Verfahren nach einem der vorhergehenden Ansprüche, **gekennzeichnet durch** die nachfolgenden Schritte,

   - Bereitstellung eines im Wesentlichen flachen und/oder plattenförmig ausgeführten porösen (Dental-)Keramikrohlings (100),
   - Versehen einer oder mehrerer Oberflächen (112, 116) des Keramikrohlings (100) mit einer Isolierung und/oder Abdichtung, wobei der Keramikrohling passgenau innerhalb einer wasser-/luftundurchlässigen Form (118), insbesondere einer Silikonform platziert wird, sodass mindestens eine Oberfläche (114) des Keramikrohlings frei zugänglich ist,
   - Beladen der frei zugänglichen Oberfläche (114) des Keramikrohlings (100) mit Farbpigmenten (101, 102), wobei die Farbpigmente (101, 102) in einer flüssigen Lösung enthalten sind,
   - Platzieren des Keramikrohlings (100) innerhalb einer Umgebung (108) deren Umgebungsparameter (106), insbesondere die Luftfeuchtigkeit und/oder der Druck und/oder die Temperatur, regelbar sind, wobei die frei zugängliche Oberfläche (114) des Keramikrohlings (100) mit der Umgebung (108) in Verbindung steht,
   - Steuerung der Verteilung der Farbpigmente (101, 102) innerhalb des Keramikrohlings (100), wobei mindestens ein Umgebungsparameter (108), insbesondere die Luftfeuchtigkeit und/oder der Druck und/oder die Temperatur zur Erzeugung eines Umgebungsparametergradienten (110) zwischen der frei zugänglichen Oberfläche (114) und der einen oder den mehreren isolierten und/oder abgedichteten Oberflächen (112, 116) des Keramikrohlings (100) geregelt wird.

13.  Keramikrohling (100), geeignet zur Herstellung von Zahnersatz mittels einer CAD/CAM-Fräsmaschine, insbesondere in einem Verfahren, nach einem der Ansprüche 1-12 hergestellt, **gekennzeichnet durch** eine Farbpigment-Verteilung (101, 102), die mittels Umgebungsparametergradienten (110) steuerbar ist, wobei der Keramikrohling (100) eine gleichmäßige, monochrome Färbung und/oder einen polychromen Farbverlauf und/oder einen räumlich, polychromen Farbverlauf aufweist.

14. Vorrichtung zur Beladung einer porösen Keramik (100), insbesondere eines Dental-Keramikrohlings, mit einer wässrigen Farbpigmentlösung, **dadurch gekennzeichnet, dass** die Beladungsvorrichtung (120) ein poröses und/oder schwammartiges Material umfasst.

15. Vorrichtung zur Steuerung einer gezielten Verteilung von Farbpigmenten innerhalb einer porösen Keramik (100), insbesondere eines Dental-Keramikrohlings, **dadurch gekennzeichnet, dass** die Vorrichtung zur Isolierung und/oder Abdichtung mindestens einer Oberfläche (116) und/oder mindestens eines Teils einer Oberfläche (112) der porösen Keramik (100) geeignet ist, wobei eine andere Oberfläche (114) und/oder ein anderer Teil einer Oberfläche zur Beaufschlagung mit regelbaren Umgebungsparametern (106) frei zugänglich ist.

## Fig. 1

| 1a | Keramik bereitstellen |

⇩

| 2b | Keramikrohling in (Konvektions)-Gehäuse einlegen |

⇩

| 3c | Konvektionsdruckhaltende Beflutung, Kapillardruckbeflutung, Befüllung der Porositäten |

⇩

| 4d | Konvektionsgesteuerte Verteilung der Komponenten |

⇩

| 5e | gesteuerter Flüssigkeitsentzug bis zum Ende der Konvektionsbewegung im Konvektionsgehäuse |

⇩

| 6f | Endtrocknung des Keramikrohlings |

⇩

| 7g | CAD/CAM-Bearbeitung |

⇩

| 8h | (End)-Sinterung |

Fig. 2

Fig. 3

Fig. 4

Fig. 5

Fig. 6

Fig. 7

Fig. 8

Fig. 9

Fig. 10

Fig. 11

Fig. 12

Europäisches Patentamt
European Patent Office
Office européen des brevets

## EUROPÄISCHER RECHERCHENBERICHT

Nummer der Anmeldung

EP 16 16 9366

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | G W LIU ET AL: "Fabrication of coloured zirconia ceramics by infiltrating water debound injection moulded green body", ADVANCES IN APPLIED CERAMICS: STRUCTURAL, FUNCTIONAL ANDBIOCERAMICS, Bd. 110, Nr. 1, 2011, Seiten 58-62, XP055253045, GB ISSN: 1743-6753, DOI: 10.1179/174367509X12502621261532 * "Introduction"; "Sample preparation"; "Results and discussion"; Abbildungen 2-5 *<br>----- | 1,3,10, 13 | INV.<br>A61K6/02<br>C04B41/00<br>C04B41/45<br>C04B41/89<br>C04B41/52<br>A61C13/083<br>B05C1/00<br>A61C13/00<br>A61K6/00<br><br>ADD.<br>C04B111/00<br>C04B111/82 |
| X<br><br>Y | WO 2014/206439 A1 (WDT WOLZ DENTAL TECHNIK GMBH [DE]) 31. Dezember 2014 (2014-12-31) * Absätze [0012], [0015], [0016], [0018], [0019], [0026], [0033], [0047], [0052] - [0056], [0060]; Abbildungen 1,4 *<br>----- | 1-5, 7-13,15<br>6 | |
| X | WO 2013/170705 A1 (SHENZHEN UPCERA CO LTD [CN]; LIAONING UPCERA CO LTD [CN]) 21. November 2013 (2013-11-21) * Beispiel 2 *<br>----- | 14 | RECHERCHIERTE SACHGEBIETE (IPC)<br><br>A61K<br>C04B<br>A61C<br>B05C |
| Y | DE 11 11 775 B (WIENAND ZAHNFAB) 27. Juli 1961 (1961-07-27) * Spalte 3, Zeile 55 - Spalte 4, Zeile 27 *<br>----- | 6 | |

Der vorliegende Recherchenbericht wurde für alle Patentansprüche erstellt

| Recherchenort | Abschlußdatum der Recherche | Prüfer |
|---|---|---|
| München | 13. Januar 2017 | Bonneau, Sébastien |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 16 16 9366

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-01-2017

| Im Recherchenbericht angeführtes Patentdokument | Datum der Veröffentlichung | Mitglied(er) der Patentfamilie | Datum der Veröffentlichung |
|---|---|---|---|
| WO 2014206439 A1 | 31-12-2014 | EP 3013278 A1<br>WO 2014206439 A1 | 04-05-2016<br>31-12-2014 |
| WO 2013170705 A1 | 21-11-2013 | CN 102674888 A<br>CN 103113132 A<br>EP 2850043 A1<br>KR 20150008876 A<br>US 2015122147 A1<br>WO 2013170705 A1 | 19-09-2012<br>22-05-2013<br>25-03-2015<br>23-01-2015<br>07-05-2015<br>21-11-2013 |
| DE 1111775 B | 27-07-1961 | KEINE | |

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 112009001253
- WO 2008098157 A
- WO 2013055432 A
- WO 0046168 A
- WO 2004110959 A
- DE 19904522 B4
- DE 102008026980 A1
- WO 0046168 A1
- WO 2011156602 A2
- DE 202011109956 U1
- WO 11156602 A

- EP 20130631 A
- EP 2024300 A
- WO 2014062375 A
- WO 0209612 A
- US 9212065 B2
- DE 202009018724
- EP 2359771 A
- EP 1859757 A
- US 9119696 B
- EP 2919771 A
- DE 102008026980